# EUROPEAN PATENT SPECIFICATION

(11) **EP 2 303 185 B1**
(45) Date of publication and mention of the grant of the patent: **03.12.2014**
(21) Application number: 09767869.2
(22) Date of filing: 19.06.2009
(51) Int. Cl.: A61F 2/82, A61L 31/04, A61L 31/14

(54) **STENT FABRICATION VIA TUBULAR CASTING PROCESSES**
STENTHERSTELLUNG MIT RÖHRENFÖRMIGEN GIESSVERFAHREN
FABRICATION DE STENT PAR DES PROCÉDÉS DE COULÉE TUBULAIRE

(30) Priority: 20.06.2008 US 143659
(43) Date of publication of application: 06.04.2011
(60) Divisional application: 14174820.2
(73) Proprietor: Amaranth Medical PTE, Singapore Science Park II, Singapore 117610 (SG)
(72) Inventor: RAMZIPOOR, Kamal, Fremont, CA 94539 (US); CHIA, Alfred, N. K., Singapore 550401 (SG); WANG, Liwei, Singapore 643656 (SG); LEE, Chang Yeul, Redwood City, CA 94063 (US)
(74) Representative: Price, Nigel John King
(86) International application number: PCT/US2009/048030
(87) International publication number: WO 2009/155560

(56) References cited:
- EP-A1- 1 977 721
- WO-A2-2008/089434
- US-A1- 2006 259 133
- US-A1- 2007 202 046
- US-A1- 2007 283 552
- US-A1- 2008 103 584

## Description

### FIELD OF THE INVENTION

The present invention relates generally to manufacturing processes for forming or creating devices which are implantable within a patient, such as medical devices. More particularly, the present invention relates to methods and processes for forming or creating tubular substrates which may be further processed to create medical devices having various geometries suitable for implantation within a patient.

### BACKGROUND OF THE INVENTION

In recent years there has been growing interest in the use of artificial materials, particularly materials formed from polymers, for use in implantable devices that come into contact with bodily tissues or fluids particularly blood. Some examples of such devices are artificia1 heart valves, stents, and vascular prosthesis. Some medical devices such as implantable stents which are fabricated from a metal have been problematic in fracturing or failing after implantation. Moreover, certain other implantable devices made from polymers have exhibited problems such as increased wall thickness to prevent or inhibit fracture or failure. However, stents having reduced wall thickness are desirable particularly for treating arterial diseases.

Because many polymeric implants such as stents are fabricated through processes such as extrusion or injection molding, such methods typically begin the process by starting with an inherently weak material. In the example of a polymeric stent, the resulting stent may have imprecise geometric tolerances as well as reduced wall thicknesses which may make these stents susceptible to brittle fracture.

A stent which is susceptible to brittle fracture is generally undesirable because of its limited ability to collapse for intravascular delivery as well as its limited ability to expand for placement or positioning within a vessel. Moreover, such polymeric stents also exhibit a reduced level of strength. Brittle fracture is particularly problematic in stents as placement of a stent onto a delivery balloon or within a delivery sheath imparts a substantial amount of compressive force in the material comprising the stent. A stent made of a brittle material may crack or have a very limited ability to collapse or expand without failure. Thus, a certain degree of malleability is desirable for a stent to expand, deform, and maintain its position securely within the vessel.

Accordingly, it is desirable to produce a polymeric substrate having one or more layers which retains its mechanical strength and is sufficiently ductile so as to prevent or inhibit brittle fracture, particularly when utilized as a biocompatible and/or bioabsorbable polymeric stent for implantation within a patient body.

WO 2008/089434 A2, which is admissible only under Article 54(3) EPC, discloses methods for fabricating biodegradable prostheses. The methods comprise providing a tubular body having an initial diameter, where the tubular body is composed at least partially of a substantially amorphous, biodegradable polymer. The tubular body is heated to a temperature above its glass transition temperature and below its melting point. The tubular body is then cooled to increase the crystallinity of the polymer. Either before or after this annealing process, the tubular body may be patterned into a structure capable of radial contraction and expansion in order to provide a stent or other endoprosthesis.

US 2008/103584 A1 discloses a method of making a polymer stent with enhanced mechanical strength. The method comprises the steps of:
a.) dip-coating a mandrel with a solution comprising one or more biocompatible polymers to form a polymer tube at least one of the polymers including an iodinated contrast agent;
b.) spin-drying the polymer tube around its longitudinal axis;
c.) solvent-polishing and vacuum drying the polymer tube;
d.) repeating steps a-c until the polymer tube reaches a desired thickness;
e.) necking the polymer tube by drawing the mandrel bearing the polymer tube through one or more necking dies of decreasing diameter, wherein said necking is carried out at a temperature above the glass transition temperature of the polymer and below the melting temperature of the polymer;
f.) annealing the polymer tube with an inert gas;
g.) removing the polymer tube from the mandrel; and
h.) creating a design in said polymer tube.

### SUMMARY OF THE INVENTION

According to the present invention there is provided a method of forming a self-expanding stent scaffold from a polymeric substrate, the method being as set out in claim 1.

Additional aspects of the method are set out in the dependent claims.

A number of casting processes are hereinafter described. These processes may be utilized to develop substrates (e.g., cylindrically shaped substrates, ellipsoid shaped substrates, diamond-shaped substrates, etc.) having a relatively high level of geometric precision and mechanical strength. These polymeric substrates can then be machined using any number of processes (e.g., high-speed laser sources, mechanical machining, etc.) to create devices such as stents having a variety of geometries for implantation within a patient, such as the peripheral or coronary vasculature, etc.
An example of such a casting process is to utilize a dip-coating process. The utilization of dip-coating to create a polymeric substrate having such desirable characteristics results in substrates which are able to retain the inherent properties of the starting materials. This in turn results in substrates having relatively high radial strength, ductility and associated fatigue characteristics which are retained through any additional manufacturing processes for implantation. Additionally, dip-coating the polymeric substrate also allows for the creation of substrates having multiple layers.
The molecular weight of a polymer is typically one of the factors in determining the mechanical behavior of the polymer. With an increase in the molecular weight of a polymer, there is generally a transition from brittle to ductile failure. Ductile materials also have a comparatively higher fatigue life. A mandrel is utilized to cast or dip-coat the polymeric substrate.
In dip-coating the polymeric substrate, one or more high molecular weight biocompatible and/or bioabsorbable polymers is selected for forming upon the mandrel. The one or more polymers are dissolved in a compatible solvent in one or more corresponding containers such that the appropriate solution may be placed under the mandrel. As the substrate may be formed to have one or more layers overlaid upon one another, the substrate may be formed to have a first layer of a first polymer, a second layer of a second polymer, and so on depending upon the desired structure and properties of the substrate. Thus, the various solutions and containers may be replaced beneath the mandrel between dip-coating operations in accordance with the desired layers to be formed upon the substrate such that the mandrel may be dipped sequentially into the appropriate polymeric solution.
Parameters such as the number of times the mandrel is immersed, the sequence and direction of dipping, the duration of time of each immersion within the solution, as well as the delay time between each immersion or the drying or curing time between dips and dipping and/or withdrawal rates of the mandrel to and/or from the solution may each be controlled to result in the desired mechanical characteristics. Formation via the dip-coating process may result in a polymeric substrate having substantially less wall thickness while retaining an increased level of strength in the substrate as compared to an extruded or injection-molded polymeric structure.
The immersion times as well as drying times may be uniform between each immersion or they may be varied as determined by the desired properties of the resulting substrate. Moreover, the substrate may be placed in an oven or dried at ambient temperature between each immersion or after the final immersion to attain a predetermined level of crystals, e.g., 20% to 40%, and a level of amorphous polymeric structure, e.g., 60% to 80%. Each of the layers overlaid upon one another during the dip-coating process are tightly adhered to one another and the wall thicknesses and mechanical properties of each polymer are retained in their respective layer with no limitation on the molecular weight and/or crystalline structure of the polymers utilized.
Dip-coating can be used to impart an orientation between layers (e.g., linear orientation by dipping; radial orientation by spinning the mandrel; etc.) to further enhance the mechanical properties of the formed substrate. As radial strength is a desirable attribute of stent design, post-processing of the formed substrate may be accomplished to impart such attributes. Typically, polymeric stents suffer from having relatively thick walls to compensate for the lack of radial strength, and this in turn reduces flexibility, impedes navigation, and reduces arterial luminal area immediately post implantation. Post-processing may also help to prevent material creep and recoil (creep is a time-dependent permanent deformation that occurs to a specimen under stress, typically under elevated temperatures) which are problems typically associated with polymeric stents.
Once the processing has been completed on the polymeric substrate, the substrate may be further formed or machined to create a variety of device. One example includes stents created from the cast cylinder by cutting along a length of the cylinder to create a rolled stent for delivery and deployment within the patient vasculature. Another example includes machining a number of portions to create a lattice or scaffold structure which facilitates the compression and expansion of the stent.
In other variations, in forming the stent, the substrate may be first formed at a first diameter, as described herein by immersing a mandrel into at least a first polymeric solution such that at least a first layer of a biocompatible polymer substrate is formed upon the mandrel and has a first diameter defined by the mandrel. In forming the substrate, parameters such as controlling a number of immersions of the mandrel into the first polymeric solution, controlling a duration of time of each immersion of the mandrel, and controlling a delay time between each immersion of the mandrel are controlled. With the substrate initially formed, the first diameter of the substrate may be reduced to a second smaller diameter and processed to form an expandable stent scaffold configured for delivery and deployment within a vessel, wherein the stent scaffold retains one or more mechanical properties of the polymer resin such that the stent scaffold exhibits ductility upon application of a load.
With the stent scaffold formed and heat set to have an initial diameter, it may be reduced to a second delivery diameter and placed upon a delivery catheter for intravascular delivery within a patient body comprising positioning the stent having the second diameter at a target location within the vessel, expanding the stent to a third diameter that is larger than the second diameter (and possibly smaller than the initial diameter) at the target location utilizing an inflation balloon or other mechanism, and allowing the stent to then self-expand into further contact with the vessel at the target location such that the stent self-expands over time back to its initial diameter or until it is constrained from further expansion by the vessel walls.

### BRIEF DESCRIPTION OF THE DRAWINGS

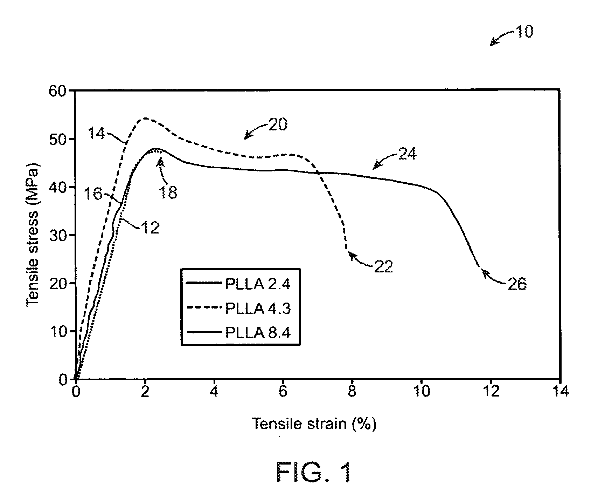

Fig. 1 illustrates a stress-strain plot of polylactic acid (PLLA) at differing molecular weights and their corresponding stress-strain values indicating brittle fracture to ductile failure.
Fig. 2A illustrates an example of a dip-coating machine which may be utilized to form a polymeric substrate having one or more layers formed along a mandrel.
Figs. 2B and 2C illustrate another example of a dip-coating assembly having one or more articulatable linkages to adjust a dipping direction of the mandrel.
Figs. 3A to 3C show respective partial cross-sectional side and end views of an example of a portion of a multi-layer polymeric substrate formed along the mandrel and the resulting substrate.
Fig. 4A illustrates an example of a resulting stress-strain plot of various samples of polymeric substrates formed by a dip-coating process and the resulting plots indicating ductile failure.
Fig. 4B illustrates another example of a stress-strain plot of additional samples formed by dip-coating along with samples incorporating a layer of BaSO₄.
Fig. 4C illustrates yet another example of a stress-strain plot of additional samples which were formed with additional layers of PLLA.
Fig. 4D illustrates an example of a detailed end view of a PLLA 8.28 substrate having a BaSO₄ layer incorporated into the substrate.
Figs. 5A and 5B illustrate perspective views of an example of a dip-coat formed polymeric substrate undergoing plastic deformation and the resulting high percentage elongation.
Fig. 6 illustrates an example of an additional forming procedure where a formed polymeric substrate may be expanded within a molding or forming tube to impart a circumferential orientation into the substrate.
Fig. 7 illustrates another example of an additional forming procedure where a formed polymeric substrate may be rotated to induce a circumferentially-oriented stress value to increase the radial strength of the substrate.
Fig. 8 illustrates a side view of a "y"-shaped mandrel which may be utilized to form a bifurcated stent via the dip coating process.
Fig. 9 illustrates a side view of another "Y"-shaped mandrel which may be utilized to form a bifurcated stent where each secondary branching member is angled with respect to one another.
Fig. 10 illustrates a side view of yet another mandrel which defines a protrusion or projection for forming a stent having an angled access port.
Fig. 11 illustrates a side view of yet another mandrel which may be used to form a stent which is tapered along its length.
Fig. 12 illustrates a side view of yet another mandrel which defines depressions or features for forming a substrate having a variable wall thickness.
Fig. 13 illustrates a perspective view of one example of a rolled sheet stent which may be formed with the formed polymeric substrate.
Fig. 14 illustrates a side view of another example of a stent machined via any number of processes from the resulting polymeric substrate.
Figs. 15 and 16 show examples of stent designs, respectively, which are optimized to take advantage of the inherent material properties of the formed polymeric substrate.
Figs. 17A to 17F illustrate side views of another example of how a stent formed from a polymeric substrate may be delivered and deployed initially via balloon expansion within a vessel and then allowed to self-expand further in diameter to its initial heat set diameter.

### DETAILED DESCRIPTION OF THE INVENTION

In manufacturing implantable devices from polymeric materials such as biocompatible and/or biodegradable polymers, a number of casting processes described herein may be utilized to develop substrates, e.g., cylindrically shaped substrates, having a relatively high level of geometric precision and mechanical strength. These polymeric substrates can then be machined using any number of processes (e.g., high-speed laser sources, mechanical machining, etc.) to create devices such as stents having a variety of geometries for implantation within a patient, such as the peripheral or coronary vasculature, etc.

An example of such a casting process is to utilize a dip-coating process. The utilization of dip-coating to create a polymeric substrate having such desirable characteristics results in substrates which are able to retain the inherent properties of the starting materials. This in turn results in substrates having a relatively high radial strength which is mostly retained through any additional manufacturing processes for implantation. Additionally, dip-coating the polymeric substrate also allows for the creation of substrates having multiple layers. The multiple layers may be formed from the same or similar materials or they may be varied to include any number of additional agents, such as one or more drugs for treatment of the vessel, as described in further detail below. Moreover, the variability of utilizing multiple layers for the substrate may allow one to control other parameters, conditions, or ranges between individual layers such as varying the degradation rate between layers while maintaining the intrinsic molecular weight and mechanical strength of the polymer at a high level with minimal degradation of the starting materials.

Because of the retention of molecular weight and mechanical strength of the starting materials via the casting or dip-coating process, polymeric substrates may be formed which enable the fabrication of devices such as stents with reduced wall thickness which is highly desirable for the treatment of arterial diseases. Furthermore these processes may produce structures having precise geometric tolerances with respect to wall thicknesses, concentricity, diameter, etc.

One mechanical property in particular which is generally problematic with, e.g., polymeric stents formed from polymeric substrates, is failure via brittle fracture of the device when placed under stress within the patient body. It is generally desirable for polymeric stents to exhibit ductile failure under an applied load rather via brittle failure, especially during delivery and deployment of a polymeric stent from an inflation balloon or constraining sheath, as mentioned above. Percent (%) ductility is generally a measure of the degree of plastic deformation that has been sustained by the material at fracture. A material that experiences very little or no plastic deformation upon fracture is brittle.

The molecular weight of a polymer is typically one of the factors in determining the mechanical behavior of the polymer. With an increase in the molecular weight of a polymer, there is generally a transition from brittle to ductile failure. An example is illustrated in the stress-strain plot **10** which illustrate the differing mechanical behavior resulting from an increase in molecular weight. The stress-strain curve **12** of a sample of polylactic acid (PLLA) 2.4 shows a failure point **18** having a relatively low tensile strain percentage at a high tensile stress level indicating brittle failure. A sample of PLLA 4.3, which has a relatively higher molecular weight than PLLA 2.4, illustrates a stress-strain curve **14** which has a region of plastic failure **20** after the onset of yielding and a failure point **22** which has a relatively lower tensile stress value at a relatively higher tensile strain percentage indicating a degree of ductility. Yield occurs when a material initially departs from the linearity of a stress-strain curve and experiences an elastic-plastic transition.

A sample of PLLA 8.4, which has yet a higher molecular weight than PLLA 4.3, illustrates a stress-strain curve **16** which has a longer region of plastic failure **24** after the onset of yielding. The failure point **26** also has a relatively lower tensile stress value at a relatively higher tensile strain percentage indicating a degree of ductility. Thus, a high-strength tubular material which exhibits a relatively high degree of ductility may be fabricated utilizing polymers having a relatively high molecular weight (e.g., PLLA 8.4, PLLA with 8.28 IV, etc.). Such a tubular material may be processed via any number of machining processes to form an implantable device such as a stent which exhibits a stress-strain curve which is associated with the casting or dip-coating process described herein. The resultant device can be subjected to relatively high levels of strain without fracturing.

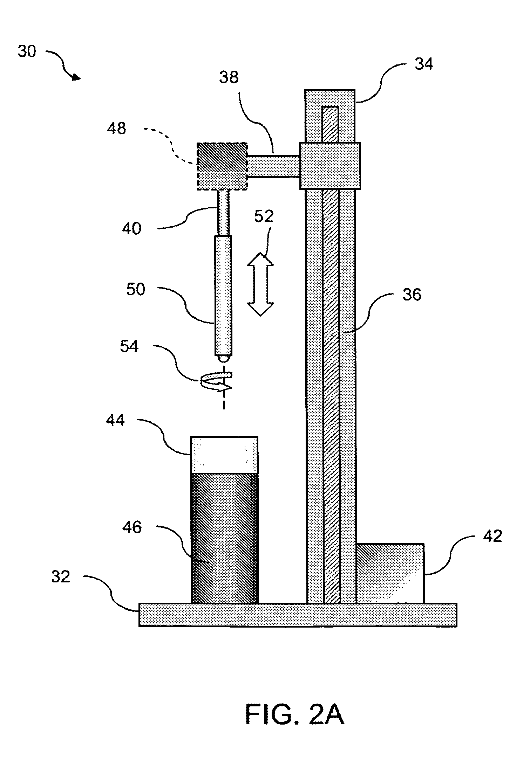

An example of a mandrel which may be utilized to cast or dip-coat the polymeric substrate is illustrated in the side view of Fig. 2A. Generally, dip coating assembly **30** may be any structure which supports the manufacture of the polymeric substrate in accordance with the description herein. A base **32** may support a column **34** which houses a drive column **36** and a bracket arm **38.** Motor **42** may urge drive column **36** vertically along column **34** to move bracket arm **38** accordingly. Mandrel **40** may be attached to bracket arm **38** above container **44** which may be filled with a polymeric solution **46** (e.g., PLLA, PLA, PLGA, etc.) into which mandrel **40** may be dipped via a linear motion **52.** The one or more polymers may be dissolved in a compatible solvent in one or more corresponding containers **44** such that the appropriate solution may be placed under mandrel **40.** An optional motor **48** may be mounted along bracket arm **38** or elsewhere along assembly **30** to impart an optional rotational motion **54** to mandrel **40** and the substrate **50** formed along mandrel **40** to impart an increase in the circumferential strength of substrate **50** during the dip-coating process, as described in further detail below.

The assembly **30** may be isolated on a vibration-damping or vibrationally isolated table to ensure that the liquid surface held within container **44** remains completely undisturbed to facilitate the formation of a uniform thickness of polymer material along mandrel **40** and/or substrate **50** with each deposition The entire assembly **30** or just a portion of the assembly such as the mandrel **40** and polymer solution may be placed in an inert environment such as a nitrogen gas environment while maintaining a very low relative humidity (RH) level, e.g., less than 30% RH, and appropriate dipping temperature, e.g., at least 20° C below the boiling point of the solvent within container **44** so as to ensure adequate bonding between layers of the dip-coated substrate. Multiple mandrels may also be mounted along bracket arm **38** or directly to column **34.**

Various drying methods may be utilized, e.g., convection, infrared, or other conventional drying techniques within a controlled environment are generally desirable as high humidity levels with high temperatures can induce hydrolysis which affects the crystallinity level and mechanical properties of the substrates during drying. For instance, PLA 8.4 substrates have a percentage of crystallinity level between, e.g., 20% to 40% or more particularly between 27% to 35%, which generally exhibit good ductility during tensile tests. If the substrates have a crystallinity that approaches 60% (which is typically the crystallinity of resin), the substrates will generally exhibit brittle failure.

Convection drying may be typically employed to uniformly heat and dry the substrates to a residual solvent level of, e.g., less than 100 ppm, while vacuum drying and/or infrared drying can be employed to shorten or reduce the typical drying time of 10 or up to 40 days depending on type of polymers used. Infrared drying can be employed to dry the surface layers at a temperature which is higher than a drying temperature of the inner layers which may contain heat sensitive drugs. In this case, the drugs within the inner layers are prevented or inhibited from degrading within the matrix. Moreover, infrared drying may prevent or inhibit the inner layers from thermal degradation if a different polymer of different glass transition temperature is used whereas convection drying for such a combination substrate may be less desirable. Generally, the drying temperature maybe performed at 5° to 10 ° C below or higher than the glass transition temperature.

The mandrel **40** may be sized appropriately and define a cross-sectional geometry to impart a desired shape and size to the substrate **50.** Mandrel **40** may be generally circular in cross section although geometries may be utilized as desired. In one example, mandrel **40** may define a circular geometry having a diameter ranging from 1 mm to 20 mm to form a polymeric substrate having a corresponding inner diameter. Moreover, mandrel **40** may be made generally from various materials which are suitable to withstand dip-coating processes, e.g., stainless steel, copper, aluminum, silver, brass, nickel, titanium, etc. The length of mandrel **40** that is dipped into the polymer solution may be optionally limited in length by, e.g., 50 cm, to ensure that an even coat of polymer is formed along the dipped length of mandrel **40** to limit the effects of gravity during the coating process. Mandrel **40** may also be made from a polymeric material which is lubricious, strong, has good dimensional stability, and is chemically resistant to the polymer solution utilized for dip-coating, e.g., fluoropolymers, polyacetal, polyester, polyamide, polyacrylates, etc.

Mandrel **40** may be made alternatively from a shape memory material, such as a shape memory polymer (SMP) or a shape memory alloy, to assist in the removal of a substrate **50** from the mandrel **40** by inducing a temporary shape of a uniform tubular form in the mandrel **40** during dipping. Additionally and/or alternatively, a layer of SMP may be utilized as a layer for dip coating substrate **50.** After drying, the substrate **50** and mandrel **40** maybe subjected to temperature change, T > T_{g} by 5° to 10 °C to induce a small deformation of less than 5% in the mandrel **40** to assist in the removal of the substrate **50** and/or for delaminating the SMP layer to further assist in removing the substrate **50.** The mandrel **40** may be comprised of various shape memory alloys, e.g., Nickel-Titanium, and various SMPs may comprise, e.g., physically cross-linked polymers or chemically cross-linked polymers etc. Examples of physically cross-linked polymers may include polyurethanes with ionic or mesogenic components made by prepolymer methods. Other block copolymers which may also be utilized may include, e.g., block copolymers of polyethyleneterephrhalate (PET) and polyethyleneoxide (PEO), block copolymers containing polystyrene and poly(1,4-butadiene), ABA triblock copolymer made from poly(2-methyl-2-oxazoline) and poly(Tetrahydrofuran), etc.

Moreover, mandrel **40** may be made to have a smooth surface for the polymeric solution to form upon. In other variations, mandrel **40** may define a surface that is coated with a material such as polytetrafluroethylene to enhance removal of the polymeric substrate formed thereon. In yet other variations, mandrel **40** may be configured to define any number of patterns over its surface, e.g., either over its entire length or just a portion of its surface, that can be mold-transferred during the dip-coating process to the inner surface of the first layer of coating of the dip-coated substrate tube. The patterns may form raised or depressed sections to form various patterns such as checkered, cross-hatched, cratered, etc. that may enhance endothelialization with the surrounding tissue after the device is implanted within a patient, e.g., within three to nine months of implantation.

The direction that mandrel **40** is dipped within polymeric solution **46** may also be alternated or changed between layers of substrate **50.** In forming substrates having a length ranging from, e.g., 1 cm to 40 on or longer, substrate **50** may be removed from mandrel **40** and replaced onto mandrel **40** in an opposite direction before the dipping process is continued. Alternatively, mandrel **40** may be angled relative to bracket arm **38** and/or polymeric solution **46** during or prior to the dipping process.

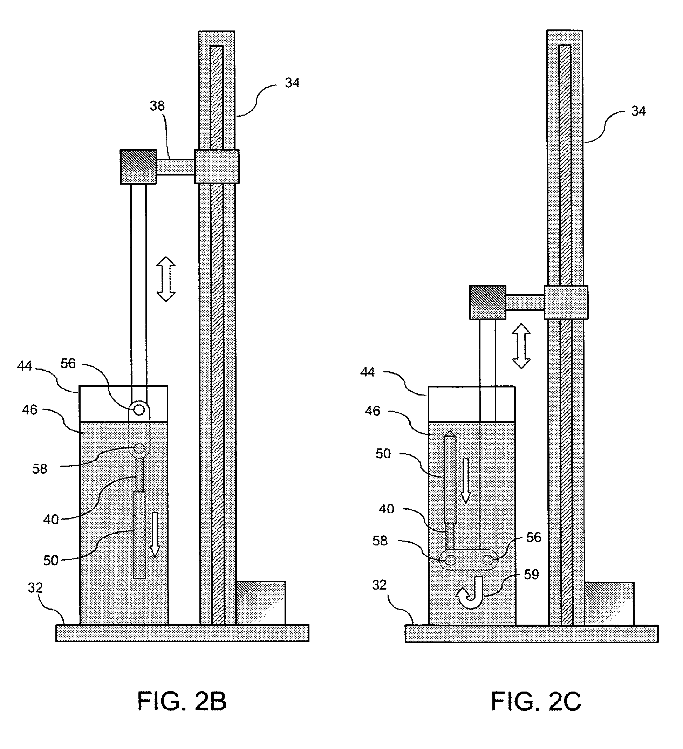

This may also be accomplished in yet another variation by utilizing a dipping assembly as illustrated in Figs. 2B and 2C to achieve a uniform wall thickness throughout the length of the formed substrate **50** per dip. For instance, after 1 to 3 coats are formed in a first dipping direction, additional layers formed upon the initial layers may be formed by dipping mandrel **40** in a second direction opposite to the first dipping direction, e.g., angling the mandrel **40** anywhere up to 180° from the first dipping direction. This may be accomplished in one example through the use of one or more pivoting linkages **56, 58** connecting mandrel **40** to bracket arm **38,** as illustrated. The one or more linkages **56, 58** may maintain mandrel **40** in a first vertical position relative to solution **46** to coat the initial layers of substrate **50,** as shown in Fig. 2B. Linkages **56, 58** may then be actuated to reconfigure mandrel **40** from its first vertical position to a second vertical position opposite to the first vertical position, as indicated by direction **59** in Fig. 2C. With repositioning of mandrel **40** complete, the dipping process may be resumed by dipping the entire linkage assembly along with mandrel **40** and substrate **50.** In this manner, neither mandrel **40** nor substrate **50** needs to be removed and thus eliminates any risk of contamination. Linkages **56, 58** may comprise any number of mechanical or electromechanical pivoting and/or rotating mechanisms as known in the art.

Dipping mandrel **40** and substrate **50** in different directions may also enable the coated layers to have a uniform thickness throughout from its proximal end to its distal end to help compensate for the effects of gravity during the coating process. These values are intended to be illustrative and are not intended to be limiting in any manner. Any excess dip-coated layers on the linkages **56, 58** may simply be removed from mandrel **40** by breaking the layers. Alternating the dipping direction may also result in the polymers being oriented alternately which may reinforce the tensile strength in the axial direction of the dip coated tubular substrate **50.**

With dip-coating assembly **30,** one or more high molecular weight biocompatible and/or bioabsorbable polymers may be selected for forming upon mandrel **40.** Examples of polymers which may be utilized to form the polymeric substrate may include, but is not limited to, polyethylene, polycarbonates, polyamides, polyesteramides, polyetheretherketone, polyacetals, polyketals, polyurethane, polyolefin, or polyethylene terephthalate and degradable polymers, for example, polylactide (PLA) including poly-L-lactide (PLLA), poly (DL-Lactide), poly-glycolide (PGA), poly(lactide-co-glycolide) (PLGA) or polycaprolactone, caprolactones, polydioxanones, polyanhydrides, polyorthocarbonates, polyphosphazenes, chitin, chitosan, poly(amino acids), and polyorthoesters, and copolymers, terpolymers and combinations and mixtures thereof.

Other examples of suitable polymers may include synthetic polymers, for example, oligomers, homopolymers, and co-polymers, acrylics such as those polymerized from methyl cerylate, methyl methacrylate, acryli acid, methacrylic acid, acrylamide, hydroxyethy acrylate, hydroxyethyl methacrylate, glyceryl scrylate, glyceryl methacrylate, methacrylamide and ethacrylamide; vinyls such as styrene, vinyl chloride, binaly pyrrolidone, polyvinyl alcohol, and vinyls acetate; polymers formed of ethylene, propylene, and tetrfluoroethylene. Further examples may include nylons such as polycoprolactam, polylauryl lactam, polyjexamethylene adipamide, and polyexamethylene dodecanediamide, and also polyurethanes, polycarbonates, polyamides, polysulfones, poly(ethylene terephthalate), polyactic acid, polyglycolic acid, polydimethylsiloxanes, and polyetherketones.

Examples of biodegradable polymers which can be used for dip-coating process are polylactide (PLA), polyglycolide (PGA), poly(lactide-co-glycolide) (PLGA), poly(e-caprolactone), polydioxanone, polyanhydride, trimethylene carbonate, poly(ß-hydroxybutyrate), poly(g-ethyl glutamate), poly(DTH iminocarbonate), poly(bisphenol A iminocarbonate), poly(ortho ester), polycyanoacrylate, and polyphosphazene, and copolymers, terpolymers and combinations and mixtures thereof. There are also a number of biodegradable polymers derived from natural sources such as modified polysaccharides (cellulose, chitin, chitosan, dextran) or modified proteins (fibrin, casein).

Other examples of suitable polymers may include synthetic polymers, for example, oligomers, homopolymers, and co-polymers, acrylics such as those polymerized from methyl cerylate, methyl methacrylate, acryli acid, methacrylic acid, acrylamide, hydroxyethy acrylate, hydroxyethyl methacrylate, glyceryl scrylate, glyceryl methacrylate, methacrylamide and ethacrylamide; vinyls such as styrene, vinyl chloride, binaly pyrrolidone, polyvinyl alcohol, and vinyls acetate; polymers formed of ethylene, propylene, and tetrfluoroethylene. Further examples may include nylons such as polycoprolactam, polylauryl lactam, polyjexamethylene adipamide, and polyexamethylene dodecanediamide, and also polyurethanes, polycarbonates, polyamides, polysulfones, poly(ethylene terephthalate), polyacetals, polyketals, polydimethylsiloxanes, and polyetherketones.

These examples of polymers which may be utilized for forming the substrate are not intended to be limiting or exhaustive but are intended to be illustrative of potential polymers which may be used. As the substrate may be formed to have one or more layers overlaid upon one another, the substrate may be formed to have a first layer of a first polymer, a second layer of a second polymer, and so on depending upon the desired structure and properties of the substrate. Thus, the various solutions and containers may be replaced beneath mandrel **40** between dip-coating operations in accordance with the desired layers to be formed upon the substrate such that the mandrel **40** may be dipped sequentially into the appropriate polymeric solution.

Depending upon the desired wall thickness of the formed substrate, the mandrel **40** may be dipped into the appropriate solution as determined by the number of times the mandrel **40** is immersed, the duration of time of each immersion within the solution, as well as the delay time between each immersion or the drying or curing time between dips. Additionally, parameters such as the dipping and/or withdrawal rate of the mandrel **40** from the polymeric solution may also be controlled to range from, e.g., 5 mm/min to 1000 mm/min. Formation via the dip-coating process may result in a polymeric substrate having half the wall thickness while retaining an increased level of strength in the substrate as compared to an extruded polymeric structure. For example, to form a substrate having a wall thickness of, e.g., 200 µm, built up of multiple layers of polylactic acid, mandrel **40** may be dipped between, e.g., 2 to 20 times or more, into the polymeric solution with an immersion time ranging from, e.g., 15 seconds (or less) to 240 minutes (or more. Moreover, the substrate and mandrel **40** may be optionally dried or cured for a period of time ranging from, e.g., 15 seconds (or less) to 60 minutes (or more) between each immersion. These values are intended to be illustrative and are not intended to be limiting in any manner.

Aside from utilizing materials which are relatively high in molecular weight, another parameter which may be considered in further increasing the ductility of the material is its crystallinity, which refers to the degree of structural order in the polymer. Such polymers may contain a mixture of crystalline and amorphous regions where reducing the percentage of the crystalline regions in the polymer may further increase the ductility of the material. Polymeric materials not only having a relatively high molecular weight but also having a relatively low crystalline percentage may be utilized in the processes described herein to form a desirable tubular substrate.

The following Table 1 show examples of various polymeric materials (e.g., PLLA IV 8.28 and PDLLA 96/4) to illustrate the molecular weights of the materials in comparison to their respective crystallinity percentage. The glass transition temperature, T_{g}, as well as melting temperature, Tₘ, are given as well. An example of PLLA IV 8.28 is shown illustrating the raw resin and tube form as having the same molecular weight, M_{w}, of 1.70 x 10⁶ gram/mol. However, the crystallinity percentage of PLLA IV 8.28 Resin is 61.90% while the corresponding Tube form is 38.40%. Similarly for PDLLA 96/4, the resin form and tube form each have a molecular weight, M_{w}, of 9.80 x 10⁵ gram/mol; however, the crystallinity percentages are 46.20% and 20.90%, respectively.

**Table 1. Various polymeric materials and their respective crystallinity percentages.**

| Material | T_{g} (°C) | Tₘ (°C) | Crystallinity (%) | M_{w} (gram / mol) |
|---|---|---|---|---|
| PLLA IV8.28 Resin | 72.5 | 186.4 | 61.90% | 1.70 x 10⁶ |
| PLLA IV8.28 Tubes | 73.3 | 176.3 | 38.40% | 1.70 x 10⁶ |
| PDLLA 96/4 Resin | 61.8 | 155.9 | 46.20% | 9.80 x 10⁵ |
| PDLLA 96/4 Tubes | 60.3 | 146.9 | 20.90% | 9.80 x 10⁵ |

As the resin is dip coated to form the tubular substrate through the methods described herein, the drying procedures and processing helps to preserve the relatively high molecular weight of the polymer from the starting material and throughout processing to substrate and stent formation. Moreover, the drying processes in particular may facilitate the formation of desirable crystallinity percentages, as described above. Furthermore, the molecular weight and crystallinity percentages, which define the strength of the substrate, are uniform within each layer as well as throughout the entire structure thereby creating a substrate that is isotropic in nature.

The resulting substrate, and the stent formed from the substrate, generally exhibits an equivalent strength in all directions. For example, the resulting stent may exhibit a radial strength which is equal to an axial or tangential strength of the stent. This feature may allow for the substrate and stent to handle loads imparted by the surrounding tissue at any number of angles. This may be particularly desirable in peripheral vessels such as the superficial femoral artery (SFA), where an implanted stent needs to be able to resist a complex and multi-axis loading condition. As strength in tubular polymeric structures are generally directional and in the case of stents, the radial strength is typically higher than the relative strengths in either the axial and tangential direction. Accordingly, the preservation of the starting polymer molecular weight helps to result in a stent having equivalent strength in all directions.

The isotropic property cannot be achieved by such processes as injection molding, extrusion and blow molding. The injection molding and extrusion processes induce axial strength while the blow molding process induces a circumferential orientation. As the result, stents that are fabricated using these processes have a preferential strength specific to the axis of orientation. In many stent designs, the isotropic material characteristics are advantageous since deformation of such material are more predictable and the prosthesis created from such substrates may have a more uniform distribution of stresses under loading conditions.

Aside from the crystallinity of the materials, the immersion times as well as drying times may be uniform between each immersion or they may be varied as determined by the desired properties of the resulting substrate.
Moreover, the substrate may be placed in an oven or dried at ambient temperature between each immersion or after the final immersion to attain a predetermined level of crystals, e.g., 20% to 40%, and a level of amorphous polymeric structure, e.g., 60% to 80%. Each of the layers overlaid upon one another during the dip-coating process are tightly adhered to one another and the mechanical properties of each polymer are retained in their respective layer with no limitation on the molecular weight of the polymers utilized. The dipping process also allows the operator to control molecular weight and crystallinity of the tubular structure which becomes the base for the resulting prosthesis. Depending on the molecular weight and crystallinity combination chosen, the resulting prosthesis may be able to provide high radial strength (e.g., 10 N per 1 cm length at 20% compression), withstand considerable amount of strain without fracturing (e.g., 150% strain), and exhibit high fatigue life under physiological conditions (e.g, 10 million cycles under radial pulse load).

Varying the drying conditions of the materials may also be controlled to effect desirable material parameters. The polymers may be dried at or above the glass transition temperature (e.g., 10° to 20° C above the glass transition temperature, T_{g}) of the respective polymer to effectively remove any residual solvents from the polymers to attain residual levels of less than 100 ppm, e.g., between 20 to 100 ppm. Positioning of the polymer substrate when drying is another factor which may be controlled as affecting parameters, such as geometry, of the tube. For instance, the polymer substrate may be maintained in a drying position such that the substrate tube is held in a perpendicular position relative to the ground such that the concentricity of the tubes is maintained. The substrate tube may be dried in an oven at or above the glass transition temperature, as mentioned, for a period of time ranging anywhere from, e.g., 10 days to 30 days or more. However, prolonged drying for a period of time, e.g., greater than 40 days, may result in thermal degradation of the polymer material.

A shape memory effect is induced in the polymer during drying of the substrate. In particular, a shape memory effect is induced in the polymeric tubing to set the tubular shape at the diameter that was formed during the dip-coating process. An example of this is to form a polymeric tube by a dip-coating process described herein at an outer diameter of 5 mm and subjecting the substrate to temperatures above its glass transition temperature, T_{g}. At its elevated temperature, the substrate may be elongated, e.g., from a length of 5 cm to 7 cm, while its outer diameter of 5 mm is reduced to 3 mm. Of course, these examples are merely illustrative and the initial diameter may generally range anywhere from, e.g., 3 mm to 10 mm, and the reduced diameter may generally range anywhere from, e.g., 1.5 mm to 5 mm, provided the reduced diameter is less than the initial diameter.

Once lengthened and reduced in diameter, the substrate is quenched or cooled in temperature to a sub-T_{g} level, e.g., about 20° C below its T_{g}, to allow for the polymeric substrate to transition back to its glass state. This effectively imparts a shape memory effect of self-expansion to the original diameter of the substrate. When such a tube (or stent formed from the tubular substrate) is compressed or expanded to a smaller or larger diameter and later exposed to an elevated temperature, over time the tube (or stent) may revert to its original 5 mm diameter. This post processing may also be useful for enabling self-expansion of the substrate after a process like laser cutting (e.g., when forming stents or other devices for implantation within the patient) where the substrate tube is typically heated to its glass transition temperature, T_{g}.

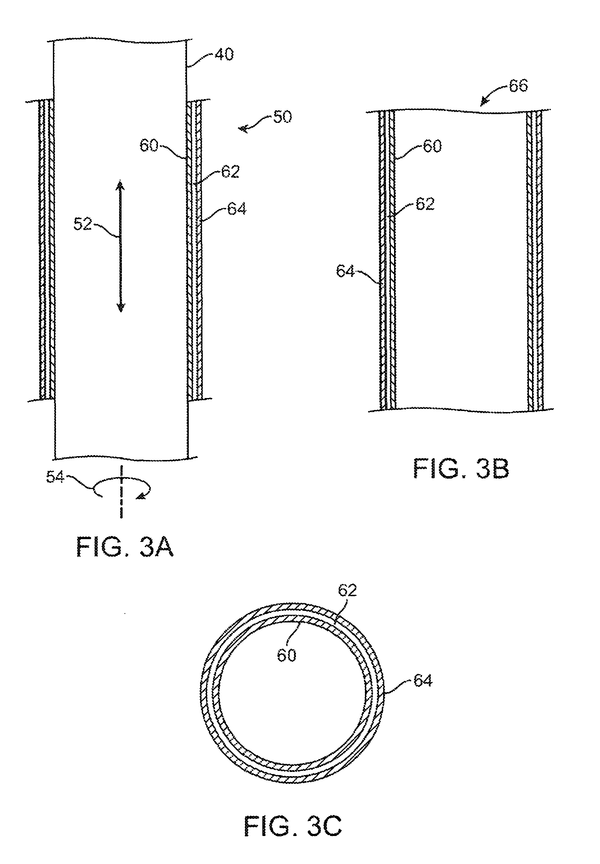

An example of a substrate having multiple layers is illustrated in Figs. 3A and 3B which show partial cross-sectional side views of an example of a portion of a multi-layer polymeric substrate formed along mandrel **40** and the resulting substrate. Substrate **50** may be formed along mandrel **40** to have a first layer **60** formed of a first polymer, e.g., poly(l-lactide). After the formation of first layer **60,** an optional second layer **62** of polymer, e.g., poly(L-lactide-co-glycolide), may be formed upon first layer **60.** Yet another optional third layer **64** of polymer, e.g., poly(d,l-lactide-co-glycolide), may be formed upon second layer **62** to form a resulting substrate defining a lumen **66** therethrough which may be further processed to form any number of devices, such as a stent. One or more of the layers may be formed to degrade at a specified rate or to elute any number of drugs or agents.

An example of this is illustrated in the cross-sectional end view of Fig. 3C, which shows an exemplary substrate having three layers **60, 62, 64** formed upon one another, as above. In this example, first layer **60** may have a molecular weight of Mₙ₁, second layer **62** may have a molecular weight of Mₙ₂, and third layer **64** may have a molecular weight of Mₙ₃. A stent fabricated from the tube may be formed such that the relative molecular weights are such where Mₙ₁ > Mₙ₂ > Mₙ₃ to achieve a preferential layer-by-layer degradation through the thickness of the tube beginning with the inner first layer **60** and eventually degrading to the middle second layer **62** and finally to the outer third layer **64** when deployed within the patient body. Alternatively, the stent may be fabricated where the relative molecular weights are such where Mₙ₁ < Mₙ₂ < Mₙ₃ to achieve a layer-by-layer degradation beginning with the outer third layer **64** and degrading towards the inner first layer **60.** This example is intended to be illustrative and fewer than or more than three layers may be utilized in other examples. Additionally, the molecular weights of each respective layer may be altered in other examples to vary the degradation rates along different layers, if so desired.

For instance, the molecular weight of different layers can also be tailored, e.g. when the first outer layer (with the minimum molecular weight Mₙ₁) degrades to certain levels, large amounts of oligomers or monomers are formed and the degradation rates of the layers are accelerated due to these low molecular weight degradation products diffused into the layers. By selecting different polymers to form the composition of this outer layer, the time needed to trigger this accelerated degradation of the other layers may be tailored. For example, any of the layers (such as the outer layer or inner layer) may be a co-polymer of 50% PLA/50% PGA where a degradation rate of the PGA is relatively faster than a degradation rate of the PLA. Thus, a layer formed of this co-polymer may have the PGA degrade relatively faster than the PLA, which in turn accelerates the degradation of the PLA itself. Alternatively or additionally, a single layer such as the outer layer may be made from such a co-polymer where degradation of the PGA in the outer layer may accelerate not only the outer layer but also the inner layer as well. Other variations may be accomplished as well depending upon the desired degradation rate and order of degradation between differing layers.

Moreover, any one or more of the layers may be formed to impart specified mechanical properties to the substrate **50** such that the composite mechanical properties of the resulting substrate **50** may specifically tuned or designed. Additionally, although three layers are illustrated in this example, any number of layers may be utilized depending upon the desired mechanical properties of the substrate **50.**

Moreover, as multiple layers may be overlaid one another in forming the polymeric substrate, specified layers may be designated for a particular function in the substrate. For example, in substrates which are used to manufacture polymeric stents, one or more layers may be designed as load-bearing layers to provide structural integrity to the stent while certain other layers may be allocated for drug-loading or eluting. Those layers which are designated for structural support may be formed from high-molecular weight polymers, e.g., PLLA or any other suitable polymer described herein, to provide a high degree of strength by omitting any drugs as certain pharmaceutical agents may adversely affect the mechanical properties of polymers. Those layers which are designated for drug-loading may be placed within, upon, or between the structural layers.

An example of utilizing layer-specific substrates may include the incorporation of one or more bio-beneficial layers that can be used to reduce the risk of blood interaction with an internal layer of a prosthesis such as the formation of thrombosis. Representative bio-beneficial materials include, but are not limited to, polyethers such as poly(ethylene glycol), copoly(ether-esters) (e.g. PEO/PLA), polyalkylene oxides such as poly(ethylene oxide), poly(propylene oxide), poly(ether ester), polyalkylene oxalates, polyphosphazenes, phosphoryl choline, choline, poly(aspirin), polymers and co-polymers of hydroxyl bearing monomers such as hydroxyethyl methacrylate (HEMA), hydroxypropyl methacrylate (HPMA), hydroxypropylmethacrylamide, poly(ethylene glycol)acrylate (PEGA), PEG methacrylate, 2-methacryloyloxyethylphosphorylcholine (MPC) and n-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), molecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharide, elastin, chitosan, alginate, silicones, PolyActive, and combinations thereof. In some embodiments, a coating described herein can exclude any one of the aforementioned polymers. The term PolyActive refers to a block copolymer having flexible poly(ethylene glycol) and poly(butylene terephthalate) blocks (PEGT/PBT). PolyActive is intended to include AB, ABA, BAB copolymers having such segments of PEG and PBT (e.g., poly(ethylene glycol)-block-poly(butyleneterephthalate)-block poly(ethylene glycol) (PEG-PBT-PEG).

In another variation, the bio-beneficial material can be a polyether such as poly(ethylene glycol) (PEG) or polyalkylene oxide. Bio-beneficial polymers that can be used to attract endothelium cells can also be coated as this first layer. These polymers, such as NO-generating polymers which may synthesized using the following strategy: (1) dispersed non-covalently bound small molecules where the diazeniumdiolate group is attached to amines in low molecular weight compounds; (2) diazeniumdiolate groups covalently bound to pendent polymer side-chains; and (3) covalently bound diazeniumdiolate groups directly to the polymer backbone. Such polymers may use diethylamine (DEA/N2O2) and diazeniumdiolated-spermine (SPER/N2O2) as the non-covalently bound species blended into both poly(ethylene glycol) (PEG) and polycaprolactone, grafting dipropylenetriamine onto a polysaccharide and by treating polyethyleneimine (PEI) with NO to form a diazeniumdiolate NO donor covalently linked directly to the polymer backbone, and 4) NO-donor that has been utilized in developing NO-releasing polymers are S-nitrosothiols (RSNOs). (Frost et al., Biomaterials, 2005, 26(14), page 1685).

In yet another example, a relatively higher molecular weight PLLA "backbone" layer, i.e., a layer which provides structural strength to a prosthesis, may be coupled with one or more various layers of other types of polymeric materials, such as poly-ε-caprolactone (PCL) or a copolymer of PCL. The backbone layer may provide strength while the PCL layer provides overall ductility to the prosthesis. The combination of layers provides a structure having both high strength and ductility. Of course, other combinations of various materials may be combined depending upon the desired resulting characteristics. For instance, another example may include a prosthesis having an inner layer made of PCL or other elastomeric polymers with a relatively high coefficient of friction. When the prosthesis is ultimately crimped onto an intravascular delivery balloon, this relatively high friction inner layer may prevent or inhibit lateral movement of the prosthesis relative to the inflation balloon to enhance stent retention on the delivery device.

Additionally, multiple layers of different drugs may be loaded within the various layers. The manner and rate of drug release from multiple layers may depend in part upon the degradation rates of the substrate materials. For instance, polymers which degrade relatively quickly may release their drugs layer-by-layer as each successive layer degrades to expose the next underlying layer. In other variations, drug release may typically occur from a multilayer matrix via a combination of diffusion and degradation. In one example, a first layer may elute a first drug for, e.g., the first 30 to 40 days after implantation. Once the first layer has been exhausted or degraded, a second underlying layer having a second drug may release this drug for the next 30 to 40 days, and so on if so desired. In the example of Fig. 3B, for a stent (or other implantable device) manufactured from substrate **50,** layer **64** may contain the first drug for release while layer **62** may contain the second drug for release after exhaustion or degradation of layer **64.** The underlying layer **60** may omit any pharmaceutical agents to provide uncompromised structural support to the entire structure.

In other examples, rather than having each successive layer elute its respective drug, each layer **62, 64** (optionally layer **60** as well), may elute its respective drug simultaneously or at differing rates via a combination of diffusion and degradation. Although three layers are illustrated in this example, any number of layers may be utilized with any practicable combination of drugs for delivery. Moreover, the release kinetics of each drug from each layer may be altered in a variety of ways by changing the formulation of the drug-containing layer.

Examples of drugs or agents which may be loaded within certain layers of substrate **50** may include one or more antipoliferative, antineoplastic, antigenic, anti-inflammatory, and/or antirestenotic agents. The therapeutic agents may also include antilipid, antimitotics, metalloproteinase inhabitors, anti-sclerosing agents. Therapeutic agents may also include peptides, enzymes, radio isotopes or agents for a variety of treatment options. This list of drugs or agents is presented to be illustrative and is not intended to be limiting.

Similarly certain other layers may be loaded with radio-opaque substances such as platinum, gold, etc. to enable visibility of the stent under imaging modalities such as fluoroscopic imaging. Radio-opaque substances like tungsten, platinum, gold, etc. can be mixed with the polymeric solution and dip-coated upon the substrate such that the radio-opaque substances form a thin sub-micron thick layer upon the substrate. The radio-opaque substances may thus become embedded within layers that degrade in the final stages of degradation or within the structural layers to facilitate stent visibility under an imaging modality, such as fluoroscopy, throughout the life of the implanted device before fully degrading or losing its mechanical strength. Radio-opaque marker layers can also be dip-coated at one or both ends of substrate **50,** e.g., up to 0.5 mm from each respective end. Additionally, the radio-opaque substances can also be spray-coated or cast along a portion of the substrate **50** between its proximal and distil ends in a radial direction by rotating mandrel **40** when any form of radio-opaque substance is to be formed along any section of length of substrate **50.** Rings of polymers having radio-opaque markers can also be formed as part of the structure of the substrate **50.**

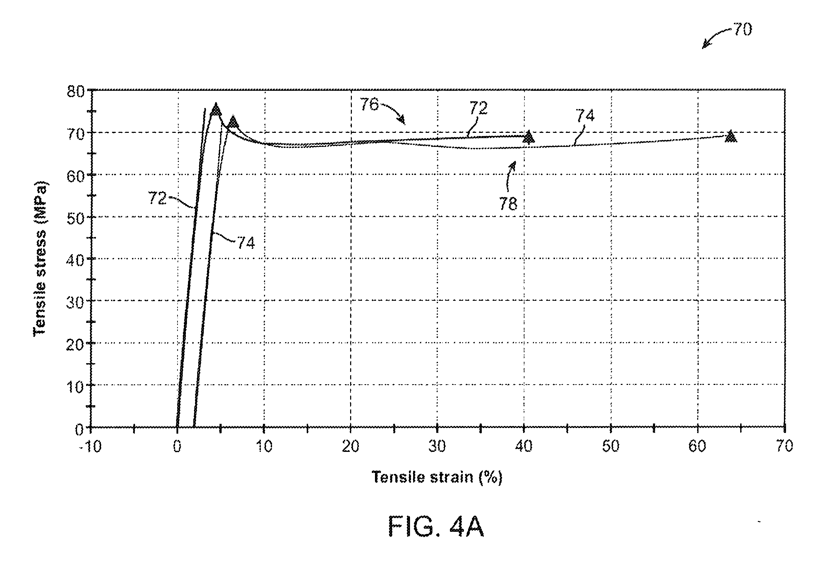

In an experimental example of the ductility and retention of mechanical properties, PLLA with Iv 8.4 (high molecular weight) was obtained and tubular substrates were manufactured utilizing the dip-coating process described herein. The samples were formed to have a diameter of 5 mm with a wall thickness of 200 µm and were comprised of 6 layers of PLLA 8.4. The mandrel was immersed 6 times into the polymeric solution and the substrates were dried or cured in an oven to obtain a 60% crystalline structure. At least two samples of tubular substrates were subjected to tensile testing and stress-strain plot **70** was generated from the stress-strain testing, as shown in Fig. 4A.

As shown in plot **70,** a first sample of PLLA 8.4 generated a stress-strain curve **72** having a region of plastic failure **76** where the strain percentage increased at a relatively constant stress value prior to failure indicating a good degree of sample ductility. A second sample of PLLA 8.4 also generated a stress-strain curve **74** having a relatively greater region of plastic failure **78** also indicating a good degree of sample ductility.

Polymeric stents and other implantable devices made from such substrates may accordingly retain the material properties from the dip-coated polymer materials. The resulting stents, for instance, may exhibit mechanical properties which have a relatively high percentage ductility in radial, torsional, and/or axial directions. An example of this is a resulting stent having an ability to undergo a diameter reduction of anywhere between 5% to 70% when placed under an external load without any resulting plastic deformation. Such a stent may also exhibit high radial strength with, e.g., 0.1 N to 5 N per one cm length at 20% deformation. Such a stent may also be configured to self-expand when exposed to normal body temperatures.

The stent may also exhibit other characteristic mechanical properties which are consistent with a substrate formed as described herein, for instance, high ductility and high strength polymeric substrates. Such substrates (and processed stents) may exhibit additional characteristics such as a percent reduction in diameter of between 5% to 70% without fracture formation when placed under a compressive load as well as a percent reduction in axial length of between 10% to 50% without fracture formation when placed under an axial load. Because of the relatively high ductility, the substrate or stent may also be adapted to curve up to 180° about a 1 cm curvature radius without fracture formation or failure. Additionally, when deployed within a vessel, a stent may also be expanded, e.g., by an inflatable intravascular balloon, by up to 5% to 80% to regain diameter without fracture formation or failure.

These values are intended to illustrate examples of how a polymeric tubing substrate and a resulting stent may be configured to yield a device with certain mechanical properties. Moreover, depending upon the desired results, certain tubes and stents may be tailored for specific requirements of various anatomical locations within a patient body by altering the polymer and/or copolymer blends to adjust various properties such as strength, ductility, degradation rates, etc.

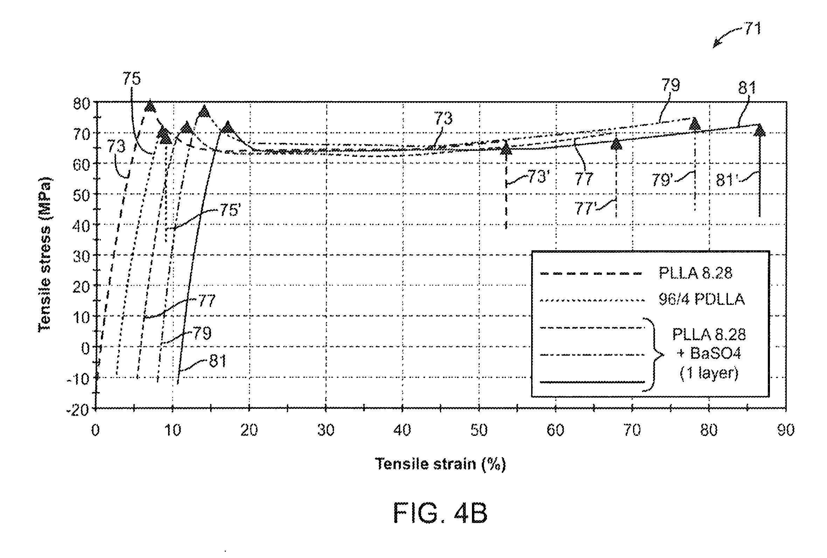

Fig. 4B illustrates a plot 71 of additional results from stress-strain testing with additional polymers. A sample of PLLA 8.28 was formed utilizing the methods described herein and tested to generate stress-strain curve 73 having a point of failure 73'. Additional samples of PLLA 8.28 each with an additional layer of BaSO₄ for radiopacity incorporated into the tubular substrate were also formed and tested. A first sample of PLLA 828 with a layer of BaSO₄ generated stress-strain curve **77** having a point of failure **77'.** A second sample of PLLA 8.28 also with a layer of BaSO₄ generated stress-strain curve **79** having a point of failure **79',** which showed a greater tensile strain than the first sample with a slightly higher tensile stress level. A third sample of PLLA 8.28 with a layer of BaSO₄ generated stress-strain curve **81** having a point of failure **81',** which was again greater than the tensile strain of the second sample, yet not significantly greater than the tensile stress level. The inclusion of BaSO₄ may accordingly improve the elastic modulus values of the polymeric substrates. The samples of PLLA 8.28 generally resulted in a load of between 100 N to 300 N at failure of the materials, which yielded elastic modulus values of between 1000 to 3000 MPa with a percent elongation of between 10% to 300% at failure.

A sample of 96/4 PDLLA was also formed and tested to generate stress-strain curve **75** having a point of failure 75' which exhibited a relatively lower percent elongation characteristic of brittle fracture. The resulting load at failure was between 100 N to 300 N with an elastic modulus of between 1000 to 3000 MPa, which was similar to the PLLA 8.28 samples. However, the percent elongation was between 10% to 40% at failure.

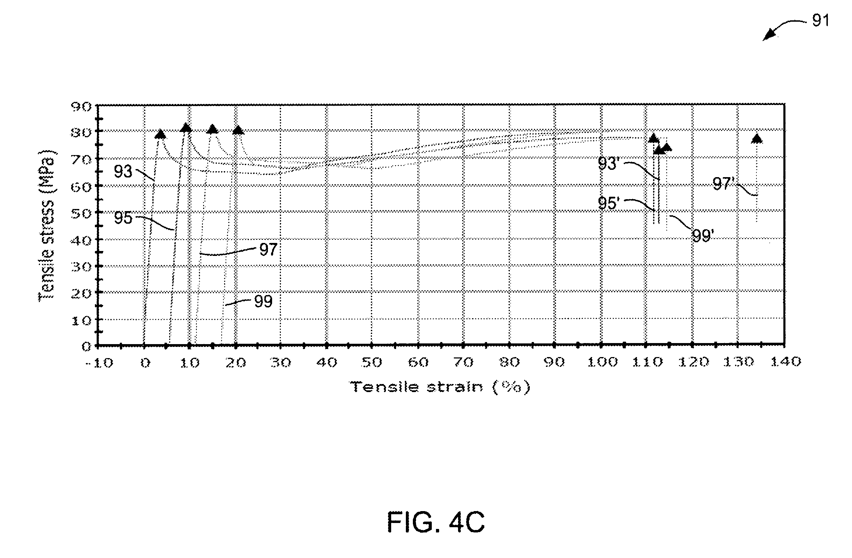

In yet another experimental example of the ductility and retention of mechanical properties, PLLA with Iv 8.28 (high molecular weight) was obtained and tubular substrates were manufactured utilizing the dip-coating process described herein. The samples were formed to have a diameter of 5 mm with a wall thickness of 200 µm and were comprised of 8 layers of PLLA 8.28. The mandrel was immersed 8 times into the polymeric solution and the substrates were dried or cured in an oven to obtain a 25% to 35% crystalline structure. At least four samples of tubular substrates were subjected to tensile testing and the stress-strain plot **91** was generated from the stress-strain testing, as shown in Fig. 4C. The following Table 2 shows the resulting stress-strain parameters for the four samples, along with the average results (Avg.) and the deviation values (Dev.).

**Table 2. Stress-strain results of PLLA 8.28.**

| No | OD (mm) | Wall thickness (mm) | Tensile stress at Yield (MPa) | Tensile strain at Yield (%) | Tensile load at break (MPa) | Tensile stress at break (MPa) | Tensile strain at break (%) | Modulus E (MPa) |
|---|---|---|---|---|---|---|---|---|
| 1 | 5.10 | 0.178 | 79.31 | 3.66 | 200.94 | 73.00 | 112.49 | 2696.00 |
| 2 | 5.09 | 0.175 | 81.70 | 3.61 | 208.84 | 77.29 | 105.71 | 2786.56 |
| 3 | 5.09 | 0.175 | 81.06 | 3.69 | 208.58 | 77.19 | 122.53 | 2692.60 |
| 4 | 5.10 | 0.177 | 80.62 | 3.73 | 202.93 | 74.09 | 97.21 | 2660.43 |
| Avg | 5.10 | 0.176 | 80.67 | 3.67 | 205.32 | 75.39 | 109.48 | 2708.90 |
| Dev | 0.01 | 0.002 | 1.01 | 0.05 | 4.00 | 2.18 | 10.71 | 54.20 |

The samples of PLLA 8.28 generally resulted in a percent elongation of between 97% to 123% at failure when placed under a 73 to 77 MPa stress load. As shown in the plot of Fig. 4C, a first sample (sample no. 1 of Table 2) of PLLA 8.28 generated a stress-strain curve **93** having a region of plastic failure **93'** where the strain percentage increased at a relatively constant stress value prior to failure indicating a good degree of sample ductility. A second sample (sample no. 2 of Table 2) of PLLA 8.28 also generated a stress-strain curve **95** having a relatively smaller region of plastic failure **95'** also indicating a good degree of sample ductility. Additional samples (sample nos. 3 and 4 of Table 2) having corresponding stress-strain curves **97, 99** and their corresponding regions of plastic failure **97', 99'** are also shown.

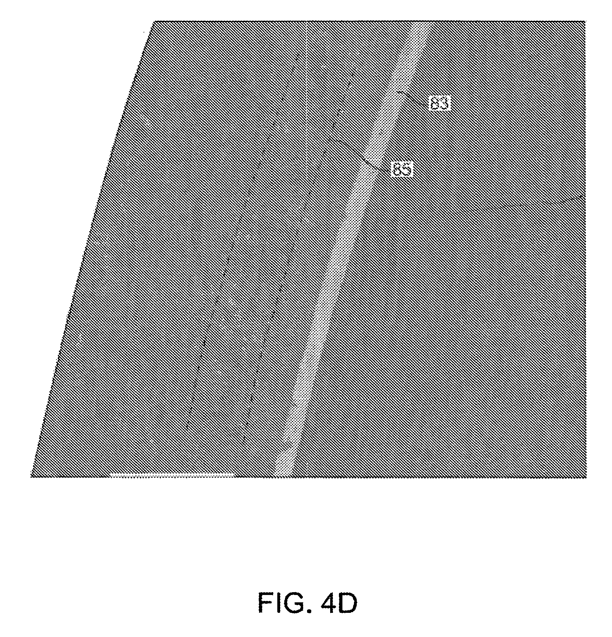

Fig. 4D illustrates an example of a detailed end view of a PLLA 8.28 substrate **83** formed with multiple dip-coated layers via a process described herein as viewed under a scanning electron microscope. This variation has a BaSO₄ layer **85** incorporated into the substrate. As described above, one or more layers of BaSO₄ may be optionally incorporated into substrate **83** to alter the elastic modulus of the formed substrate and to provide radiopacity. Additionally, the individual layers overlaid atop one another are fused to form a single cohesive layer rather than multiple separate layers as a result of the drying processes during the dipping process described herein. This results in a unitary structure which further prevents or inhibits any delamination from occurring between the individual layers.

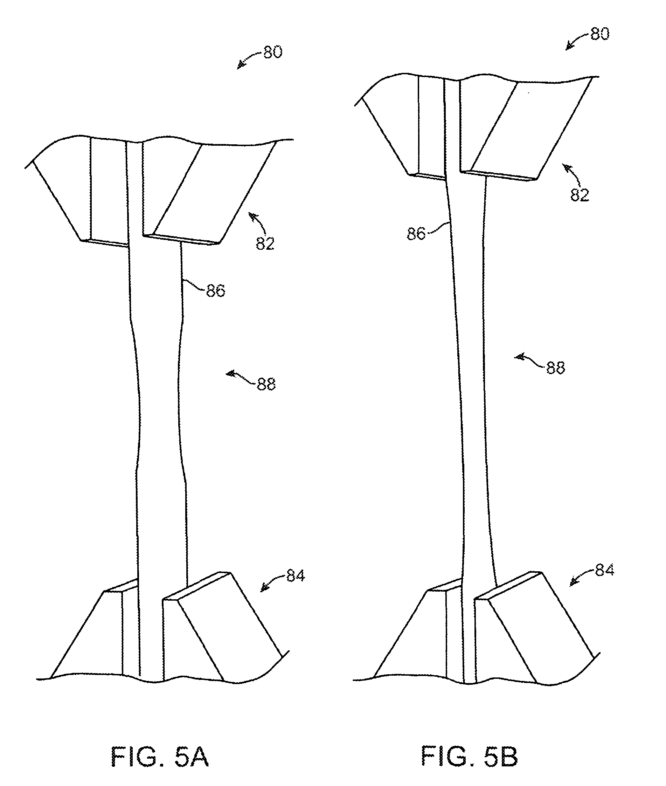

Figs. 5A and 5B illustrate perspective views of one of the samples which was subjected to stress-strain testing on tensile testing system **80.** The polymeric substrate specimen **86** was formed upon a mandrel, as described above, into a tubular configuration and secured to testing platform **82, 84.** With testing platform **82, 84** applying tensile loading, substrate specimen **86** was pulled until failure. The relatively high percentage of elongation is illustrated by the stretched region of elongation **88** indicating a relatively high degree of plastic deformation when compared to an extruded polymeric substrate. Because a polymeric substrate formed via dip-coating as described above may be reduced in diameter via plastic deformation without failure, several different stent diameters can be manufactured from a single diameter substrate tube.

Dip-coating can be used to impart an orientation between layers (e.g., linear orientation by dipping; radial orientation by spinning the mandrel; etc.) to further enhance the mechanical properties of the formed substrate. As radial strength is a desirable attribute of stent design, post-processing of the formed substrate may be accomplished to impart such attributes. Typically, polymeric stents suffer from having relatively thick walls to compensate for the lack of radial strength, and this in turn reduces flexibility, impedes navigation, and reduces arterial luminal area immediately post implantation. Post-processing may also help to prevent material creep and recoil (creep is a time-dependent permanent deformation that occurs to a specimen under stress, typically under elevated temperatures) which are problems typically associated with polymeric stents. By using a relatively high molecular weight in a range of, e.g., 259,000 g/mol to 2,120,000 g/mol, and controlling dipping parameters such as speed and temperature as well as the drying condition, the dipped substrates will have the following desirable properties: (1) high radial strength; (2) ductility; (3) malleability; and (4) isotropicity.

In further increasing the radial or circumferential strength of the polymeric substrate, a number of additional processes may be applied to the substrate after the dip-coating procedure is completed (or close to being completed). A polymer that is amorphous or that is partially amorphous will generally undergo a transition from a pliable, elastic state (at higher temperatures) to a brittle glass-like state (at lower temperature) as it transitions through a particular temperature, referred as the glass transition temperature (T_{g}). The glass transition temperature for a given polymer will vary, depending on the size and flexibility of side chains, as well as the flexibility of the backbone linkages and the size of functional groups incorporated into the polymer backbone. Below T_{g}, the polymer will maintain some flexibility, and may be deformed to a new shape. However, the further the temperature below T_{g} the polymer is when being deformed, the greater the force needed to shape it.

Moreover, when a polymer is in glass transition temperature its molecular structure can be manipulated to form an orientation in a desired direction. Induced alignment of polymeric chains or orientation improves mechanical properties and behavior of the material. Molecular orientation is typically imparted by application of force while the polymer is in a pliable, elastic state. After sufficient orientation is induced, temperature of the polymer is reduced to prevent reversal and dissipation of the orientation.

In an example not in accordance with the present invention, the polymeric substrate is heated to increase its temperature along its entire length or along a selected portion of the substrate to a temperature that is at or above the T_{g} of the polymer. For instance, for a substrate fabricated from PLLA, the substrate is heated to a temperature between 60° C to 70° C. Once the substrate has reached a sufficient temperature such that enough of its molecules have been mobilized, a force is applied from within the substrate or along a portion of the substrate to increase its diameter from a first diameter D₁ to a second increased diameter D₂ for a period of time necessary to set the increased diameter. During this setting period, the application of force induces a molecular orientation in a circumferential direction to align the molecular orientation of polymer chains to enhance its mechanical properties. The re-formed substrate may then be cooled to a lower temperature typically below T_{g}, for example, by passing the tube through a cold environment, typically dry air or an inert gas to maintain the shape at diameter D₂ and prevent dissipation of molecular orientation.

The force applied to the substrate may be generated by a number of different methods. One method is by utilizing an expandable pressure vessel placed within the substrate. Another method is by utilizing a braid structure, such as a braid made from a super-elastic or shape memory alloy like NiTi alloy, to increase in size and to apply the desirable degree of force against the interior surface of the substrate.

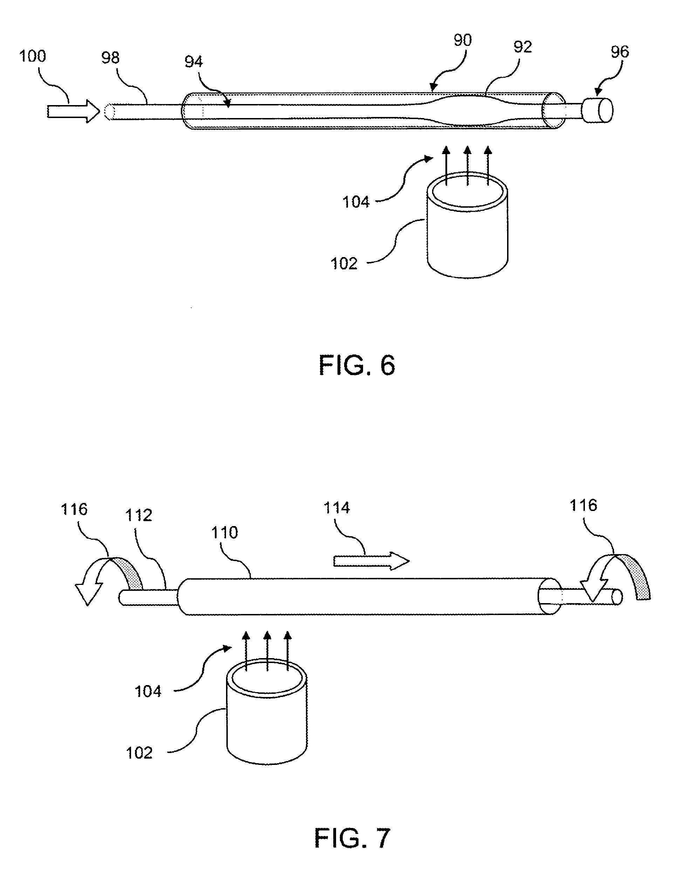

Yet another method may apply the expansion force by application of a pressurized inert gas such as nitrogen within the substrate lumen, as shown in Fig. 6, to impart a circumferential orientation in the substrate. A completed substrate, e.g., cast cylinder **94,** may be placed inside a molding tube **90** which has an inner diameter that is larger than the cast cylinder **94.** Molding tube **90** may be fabricated from glass, highly-polished metal, or polymer. Moreover, molding tube **90** may be fabricated with tight tolerances to allow for precision sizing of cast cylinder **94.**

A distal end or distal portion of cast cylinder **94** may be clamped **96** or otherwise closed and a pressure source may be coupled to a proximal end **98** of cast cylinder **94.** The entire assembly may be positioned over a nozzle **102** which applies heat **104** to either the length of cast cylinder **94** or to a portion of cast cylinder **94.** The pressurized inert gas **100,** e.g., pressured to 69 kPa to 2.7 GPa (10 to 400 psi), may be introduced within cast cylinder **94** to increase its diameter, e.g., 2 mm, to that of the inner diameter, e.g., 4 mm, of molding tube **90.** The increase in diameter of cast cylinder **94** may thus realign the molecular orientation of cast cylinder **94** to increase its radial strength and to impart a circumferential orientation in the cast cylinder **94.** Portion **92** illustrates radial expansion of the cast cylinder **94** against the inner surface of the molding tube **90** in an exaggerated manner to illustrate the radial expansion and impartation of circumferential strength. After the diameter has been increased, cast cylinder **94** may be cooled, as described above.

Once the substrate has been formed and reduced in diameter to its smaller second diameter, the stent may be processed, as described above. Alternatively, the stent may be processed from the substrate after initial formation. The stent itself may then be reduced in diameter to its second reduced diameter.

In either case, once the stent has been formed into its second reduced diameter, the stent may be delivered to a targeted location within a vessel of a patient. Delivery may be effected intravascularly utilizing known techniques with the stent in its second reduced delivery diameter positioned upon, e.g., an inflation balloon, for intravascular delivery. Once the inflation catheter and stent has been positioned adjacent to the targeted region of vessel, the stent may be initially expanded into contact against the interior surface of the vessel.

With the stent expanded into contact against the vessel wall at a third diameter which is larger than the second delivery diameter, the inflation balloon may be removed from the stent. Over a predetermined period of time and given the structural characteristics of the stent, the stent may then also self-expand further into contact against the vessel wall for secure placement and positioning.

Because thermoplastic polymers such as PLLA typically soften when heated, the cast cylinder **94** or a portion of the cast cylinder **94** may be heated in an inert environment, e.g., a nitrogen gas environment, to minimize its degradation.

Another method for post-processing a cast cylinder **110** may be seen in the example of Fig. 7 for inducing a circumferential orientation in the formed substrate. As illustrated, mandrel **112** having the cast cylinder **110** may be re-oriented into a horizontal position immediately post dip-coating before the polymer is cured. Mandrel **112** may be rotated, as indicated by rotational movement **116,** at a predetermined speed, e.g., 1 to 300 rpm, while the cylinder **110** is heated via nozzle **102.** Mandrel **112** may also be optionally rotated via motor **48** of assembly **30** to impart the rotational motion **54,** as shown above in Fig. 2. Mandrel **112** may also be moved in a linear direction **114** to heat the length or a portion of the length of the cylinder **110.** As above, this post-processing may be completed in an inert environment.

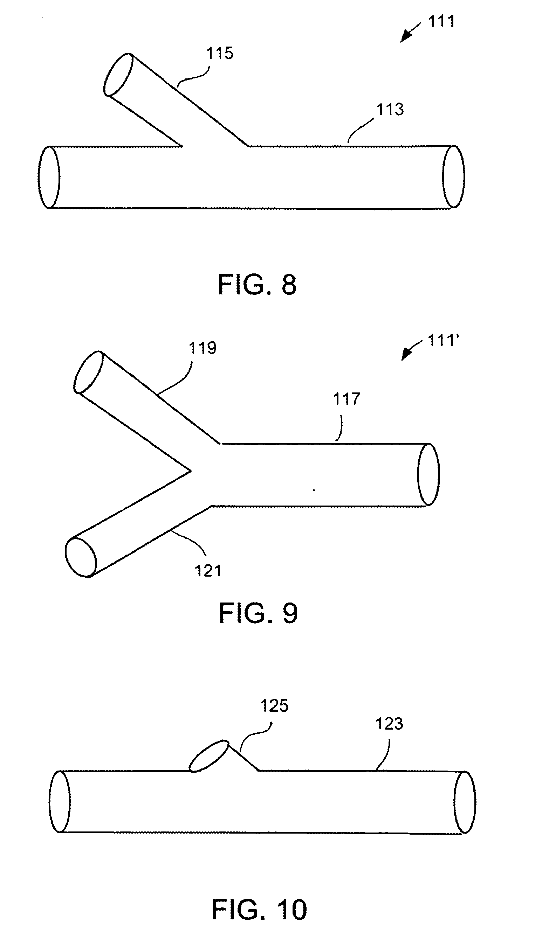

In other variations, the mandrel itself may be fabricated into alternative configurations aside from a cylindrical shape to impart these configurations directly into the substrates formed thereupon. An example is illustrated in the side view of Fig. 8 which shows a bifurcated "y"-shaped mandrel **111** comprised of an elongate primary support member **113** (having a circular, elliptical, or any other cross-sectional area, as desired) with a secondary branching support member **115** projecting at an angle from primary support member **113.** The mandrel **111** may be fabricated as a single, integral piece or from several individual portions which may be assembled and de-assembled to assist in fabricating a substrate or removing a formed substrate from the mandrel **111.** A multi-directional dipping process, such as three-dimensional dipping while rotating, as well as multi-directional curing, such as three-dimensional curing while rotating, may be utilized to form and maintain a uniform wall thickness of the substrate over the length of mandrel **111** to form an integral and uniform bifurcated substrate and subsequently a bifurcated stent scaffold.

Another variation is shown in the side view of Fig. 9 which shows a bifurcated "Y"-shaped mandrel **111'** having an elongated primary support member **117** which branches in a bifurcation into at least two secondary branching support members **119, 121** which are angled with respect to each other as well as with respect to primary support member **117.** Such a mandrel **111'** may be formed of a singular integral piece or formed from individual portions which are attached to one another for forming the substrate and removing the substrate from the mandrel **111'.**

Yet another variation is shown in the side view of Fig. 10, which shows a mandrel having a primary support member **123** with a protrusion **125** extending at an angle with respect to primary support member **123.** Protrusion **125** may just extend beyond support member **123** to form a substrate and stent scaffold which has a portal formed about protrusion **125.** A stent formed with such a portal may be commonly used for accessing a side branch vessel extending from a primary vessel.

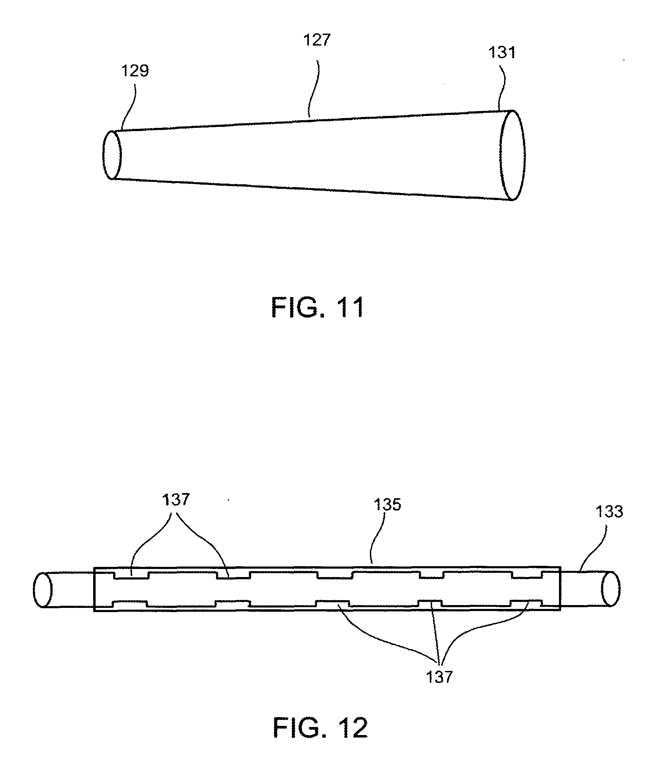

In yet another variation as illustrated in Fig. 11 for directly forming substrates (and stent scaffolds) having alternative configurations, a tapered mandrel **127** having an elongate body which tapers from a narrowed end **129** to a widened end **131** may be utilized to subsequently form tapered stent prostheses which may be implanted along vessels which taper to prevent over-stretching of the vessel and minimize any injuries. The length and angle of tapering may be adjusted along the mandrel **127** to form a substrate which is suited for a particular anatomy, if so desired. Yet another variation includes dip coating a metallic stent (such as a stainless steel or Nitinol stent) into a polymeric solution as described herein where the solution incorporates one or more drugs or radiopaque agents such as Pt/Ir, gold, or tungsten, etc. The polymeric coating can be used to deliver or elute drugs or the coating may be used to enhance radiopacity of the stent while the coated stent is able to maintain radial forces via its metallic structure.

As discussed above, another method for substrate and stent fabrication is to form a substrate having a variable wall thickness, as illustrated in the side view of Fig. 12. In this variation, a dipping mandrel **133** having one or more diameters or surface features may be utilized. The variations in diameters or features may be produced by forming one or more depressions or features **137,** e.g., peaks and valleys, along the surface of mandrel **133.** These depressions or features **137** may be uniformly or arbitrarily located along the mandrel **133.** The polymeric substrate **135** formed upon mandrel **133** utilizing the methods herein may thus be formed to have the corresponding features defined on the inner surface along its length. Thus, the resulting stent having a variable wall thickness structure may provide increased longitudinal flexibility while retaining other desirable stent qualities such as radial strength equal to or greater than existing endovascular stents.

The dipping process does not require a high temperature. The operation is typically conducted under ambient or below ambient temperatures. At such a temperature, pharmaceutical agents can be distributed into the polymer matrix without thermal effects, which tends to denature most drugs. The drug may also be protected from oxidization by an inert dipping environment and vacuum drying at a very low temperature

Alternatively and as described above, a surface of the mandrel can be formed in a pattern configured to form holes or voids (e.g., cylindrically or rectangularly shaped) into the inner layer of polymer substrate. The formed holes or voids may be formed, for instance, to have a volume of 10 - 100 µl. These structures may function as reservoirs and can be used to hold various materials for delivery into the patient (e.g., drug molecules, peptides, biological reagents, etc.) by dip coating a substrate into a reservoir containing the material to be introduced into the holds or voids where the solution has a relatively low viscosity ranging from 1.0 × 10⁻³ to 50 × 10⁻³ Pa·s. Filling of the holes or voids can also be accomplished by directly inject the eluting material into the holes or voids along the substrate. By doing so, the drugs, peptide, biological agents, etc. that are sensitive to temperature can be incorporated directly into the substrate and/or stent for release from the implanted prosthesis. In some variations, the implanted prosthesis can optionally include at least one biologically active ("bioactive") agent. The at least one bioactive agent can include any substance capable of exerting a therapeutic, prophylactic or diagnostic effect for a patient.

Examples of suitable bioactive agents include, but are not limited to, synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. The bioactive agents could be designed, e.g., to inhibit the activity of vascular smooth muscle cells. They could be directed at inhibiting abnormal or inappropriate migration and/or proliferation of smooth muscle cells to inhibit restenosis.

In other variations, optionally in combination with one or more other variations described herein, the implantable prosthesis can include at least one biologically active agent selected from antiproliferative, antineoplastic, antimitotic, anti-inflammatory, antiplatelet, anticoagulant, antifibrin, antithrombin, antibiotic, antiallergic and antioxidant substances.

An antiproliferative agent can be a natural proteineous agent such as a cytotoxin or a synthetic molecule. Examples of antiproliferative substances include, but are not limited to, actinomycin D or derivatives and analogs thereof (manufactured by Sigma-Aldrich, or COSMEGEN available from Merck) (synonyms of actinomycin D include dactinomycin, actinomycin IV, actinomycin I₁, actinomycin X₁, and actinomycin C₁); all taxoids such as taxols, docetaxel, and paclitaxel and derivatives thereof; all olimus drugs such as macrolide antibiotics, rapamycin, everolimus, structural derivatives and functional analogues of rapamycin, structural derivatives and functional analogues of everolimus, FKBP-12 mediated mTOR inhibitors, biolimus, perfenidone, prodrugs thereof, co-drugs thereof, and combinations thereof. Examples of rapamycin derivatives include, but are not limited to, 40-0-(2-hydroxy)ethyl-rapamycin (trade name everolimus from Novartis), 40-O-(2-ethoxy)ethyl-rapamycin (biolimus), 40-0-(3-hydroxy)propyl-rapamycin, 40-O-[2-(2-hydroxy)ethoxy]ethyl-rapamycin, 40-O-tetrazole-rapamycin, 40-epi-(N1-tetrazolyl)-rapamycin (zotarolimus, manufactured by Abbott Labs.), Biolimus A9 (Biosensors International, Singapore), AP23572 (Ariad Pharmaceuticals), prodrugs thereof, co-drugs thereof, and combinations thereof.

An anti-inflammatory drug can be a steroidal anti-inflammatory drug, a nonsteroidal anti-inflammatory drug (NSAID), or a combination thereof. Examples of anti-inflammatory drugs include, but are not limited to, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol, clobetasol propionate, clobetasone butyrate, clopirac, cloticasone propionate, cormethasone acetate, cortodoxone, deflazacort, desonide, desoximetasone, dexamethasone, dexamethasone acetate, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isofluprectone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof.

Alternatively, the anti-inflammatory agent can be a biological inhibitor of pro-inflammatory signaling molecules. Anti-inflammatory biological agents include antibodies to such biological inflammatory signaling molecules.

In addition, the bioactive agents can be other than antiproliferative or anti-inflammatory agents. The bioactive agents can be any agent that is a therapeutic, prophylactic or diagnostic agent. In some embodiments, such agents can be used in combination with antiproliferative or anti-inflammatory agents. These bioactive agents can also have antiproliferative and/or anti-inflammmatory properties or can have other properties such as antineoplastic, antimitotic, cystostatic, antiplatelet, anticoagulant, antifibrin, antithrombin, antibiotic, antiallergic, and/or antioxidant properties.

Examples of antineoplastics and/or antimitotics include, but are not limited to, paclitaxel (e.g., TAXOL® available from Bristol-Myers Squibb), docetaxel (e.g., Taxotere® from Aventis), methotrexate, azathioprine, vincristine, vinblastine, fluorouracil, doxorubicin hydrochloride (e.g., Adriamycin®from Pfizer), and mitomycin (e.g., Mutamycin®from Bristol-Myers Squibb).

Examples of antiplatelet, anticoagulant, antifibrin, and antithrombin agents that can also have cytostatic or antiproliferative properties include, but are not limited to, sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as ANGIOMAX (from Biogen), calcium channel blockers (e.g., nifedipine), colchicine, fibroblast growth factor (FGF) antagonists, fish oil (e.g., omega 3-fatty acid), histamine antagonists, lovastatin (a cholesterol-lowering drug that inhibits HMG-CoA reductase, brand name Mevacor® from Merck), monoclonal antibodies (e.g., those specific for platelet-derived growth factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetics, 4-amino-2,2,6,6-tetramethylpiperidine-1-oxyl (4-amino-TEMPO), estradiol, anticancer agents, dietary supplements such as various vitamins, and a combination thereof.

Examples of cytostatic substances include, but are not limited to, angiopeptin, angiotensin converting enzyme inhibitors such as captopril (e.g., Capoten® and Capozide®from Bristol-Myers Squibb), cilazapril and lisinopril (e.g., Prinivil® and Prinzide® from Merck).

Examples of antiallergic agents include, but are not limited to, permirolast potassium. Examples of antioxidant substances include, but are not limited to, 4-amino-2,2,6,6-tetramethylpiperidine-1-oxyl (4-amino-TEMPO). Other bioactive agents include anti-infectives such as antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary vasodilators; peripheral and cerebral vasodilators; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and restenoic reducing agents.

Other biologically active agents that can be used include alpha-interferon, genetically engineered epithelial cells, tacrolimus and dexamethasone.

A "prohealing" drug or agent, in the context of a blood-contacting implantable device, refers to a drug or agent that has the property that it promotes or enhances re-endothelialization of arterial lumen to promote healing of the vascular tissue. The portion(s) of an implantable device (e.g., a stent) containing a prohealing drug or agent can attract, bind, and eventually become encapsulated by endothelial cells (e.g., endothelial progenitor cells). The attraction, binding, and encapsulation of the cells will reduce or prevent the formation of emboli or thrombi due to the loss of the mechanical properties that could occur if the stent was insufficiently encapsulated. The enhanced re-endothelialization can promote the endothelialization at a rate faster than the loss of mechanical properties of the stent.

The prohealing drug or agent can be dispersed in the body of the bioabsorbable polymer substrate or scaffolding. The prohealing drug or agent can also be dispersed within a bioabsorbable polymer coating over a surface of an implantable device (e.g., a stent).

"Endothelial progenitor cells" refer to primitive cells made in the bone marrow that can enter the bloodstream and go to areas of blood vessel injury to help repair the damage. Endothelial progenitor cells circulate in adult human peripheral blood and are mobilized from bone marrow by cytokines, growth factors, and ischemic conditions. Vascular injury is repaired by both angiogenesis and vasculogenesis mechanisms. Circulating endothelial progenitor cells contribute to repair of injured blood vessels mainly via a vasculogenesis mechanism.

In some embodiments, the prohealing drug or agent can be an endothelial cell (EDC)-binding agent. In certain embodiments, the EDC-binding agent can be a protein, peptide or antibody, which can be, e.g., one of collagen type 1, a 23 peptide fragment known as single chain Fv fragment (scFv A5), a junction membrane protein vascular endothelial (VE)-cadherin, and combinations thereof. Collagen type 1, when bound to osteopontin, has been shown to promote adhesion of endothelial cells and modulate their viability by the down regulation of apoptotic pathways. S.M. Martin, et al., J. Biomed. Mater. Res., 70A:10-19 (2004). Endothelial cells can be selectively targeted (for the targeted delivery of immunoliposomes) using scFv A5. T. Volkel, et al., Biochimica et Biophysica Acta, 1663:158-166 (2004). Junction membrane protein vascular endothelial (VE)-cadherin has been shown to bind to endothelial cells and down regulate apoptosis of the endothelial cells. R. Spagnuolo, et al., Blood, 103:3005-3012 (2004).

In a particular embodiment, the EDC-binding agent can be the active fragment of osteopontin, (Asp-Val-Asp-Val-Pro-Asp-Gly-Asp-Ser-Leu-Ala-Try-Gly). Other EDC-binding agents include, but are not limited to, EPC (epithelial cell) antibodies, RGD peptide sequences, RGD mimetics, and combinations thereof.

In further embodiments, the prohealing drug or agent can be a substance or agent that attracts and binds endothelial progenitor cells. Representative substances or agents that attract and bind endothelial progenitor cells include antibodies such as CD-34, CD-133 and vegf type 2 receptor. An agent that attracts and binds endothelial progenitor cells can include a polymer having nitric oxide donor groups.

The foregoing biologically active agents are listed by way of example and are not meant to be limiting. Other biologically active agents that are currently available or that may be developed in the future are equally applicable.

In a more specific embodiment, optionally in combination with one or more other embodiments described herein, the implantable device of the invention comprises at least one biologically active agent selected from paclitaxel, docetaxel, estradiol, nitric oxide donors, super oxide dismutases, super oxide dismutase mimics, 4-amino-2,2,6,6-tetramethylpiperidine-1-oxyl (4-amino-TEMPO), tacrolimus, dexamethasone, dexamethasone acetate, rapamycin, rapamycin derivatives, 40-O-(2-hydroxy)ethyl-rapamycin (everolimus), 40-O-(2-ethoxy)ethyl-rapamycin (biolimus), 40-O-(3-hydroxy)propyl-rapam 40-O-[2-(2-hydroxy)ethoxy]ethyl-rapam 40-O-tetrazole-rapamycin, 40-epi-(N1-tetrazolyl)-rapamycin (zotarolimus), Biolimus A9 (Biosensors International, Singapore), AP23572 (Ariad Pharmaceuticals), pimecrolimus, imatinib mesylate, midostaurin, clobetasol, progenitor cell-capturing antibodies, prohealing drugs, prodrugs thereof, co-drugs thereof, and a combination thereof. In a particular embodiment, the bioactive agent is everolimus. In another specific embodiment, the bioactive agent is clobetasol.

An alternative class of drugs would be p-para-agonists for increased lipid transportation, examples include feno fibrate.

In some embodiments, optionally in combination with one or more other embodiments described herein, the at least one biologically active agent specifically cannot be one or more of any of the bioactive drugs or agents described herein.

A prosthesis described above having one or more holes or voids can also be used to treat, prevent, or ameliorate any number of medical conditions located at the downstream vessel where the vessel is too narrow to allow any device to pass. By incorporation of the controlled release of various agents, these therapeutic agents may be delivered to the diseased area to provide for a regional therapy treatment carried out without the side effects that may be observed for a systematic treatment. Some exemplary treatments include delivering chemotherapeutical agents for tumor, anti inflammatory agents for kidney chronic glomerulonephritis, blood clot preventing agents for heart small vessel disease, small vessel arterial disease, small vessel peripheral arterial disease, and peripheral pulmonary vessel disease.

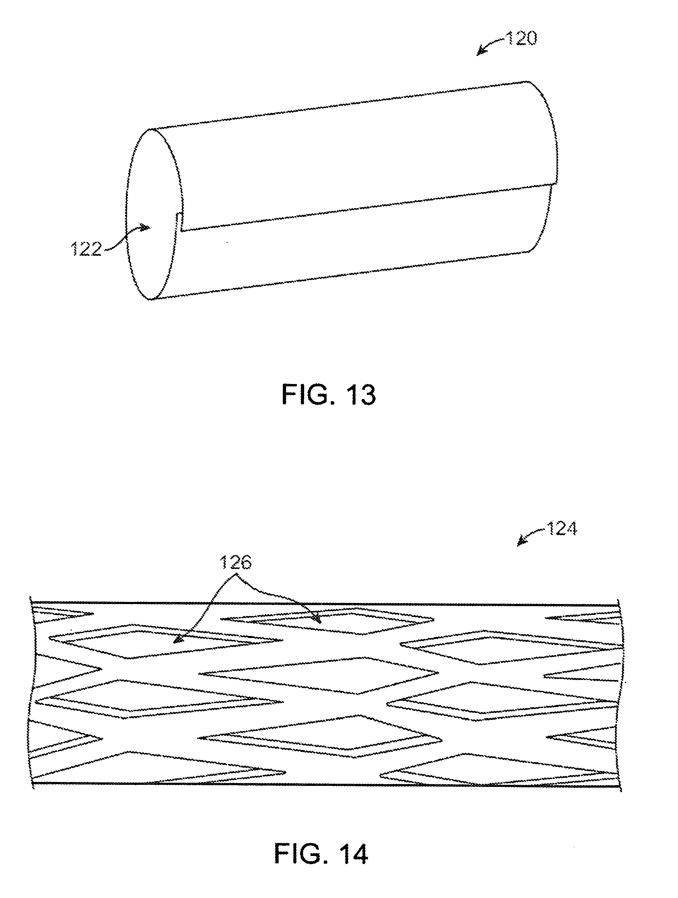

Once the processing has been completed on the polymeric substrate, the substrate may be further formed or machined to create a variety of device. One example is shown in the perspective view of Fig. 13, which illustrates rolled stent **120**. Stent **120** may be created from the cast cylinder by cutting along a length of the cylinder to create an overlapping portion **122.** The stent **120** may then be rolled into a small configuration for deployment and then expanded within the patient vasculature. Another example is illustrated in the side view of stent **124,** as shown in Fig. 14, which may be formed by machining a number of removed portions **126** to create a lattice or scaffold structure which facilitates the compression and expansion of stent **124** for delivery and deployment.

Aside from the design of stent **124** described above, other stent designs may be utilized which are particularly attuned to the physical and mechanical characteristics provided by the resulting polymeric substrate. Such stent designs may be mechanically optimized to take advantage of the ductility and strength characteristics provided by the polymeric material to result in a stent which is capable of experiencing between 10% to 80% material strain during the crimping process. For example, the starting diameter of a stent which is formed from a cured substrate may be initially at, e.g., 5 mm, and end with a crimped diameter of between, e.g., 2 to 2.8 mm. Further crimping to an even smaller diameter can increase the material strain above 100%.

Moreover, the optimized stent design may possess a relatively high fatigue life for a range of deformations by taking advantage of linear elastic properties possessed by the substrate prior to the initiation of any plastic deformation. The stent design may be modified based on physiologic conditions and materials selected so that when the stent is experiencing deformations caused by, e.g., physiologic conditions, the stent experiences material strain values that lie within the range of elastic deformation of the selected material.

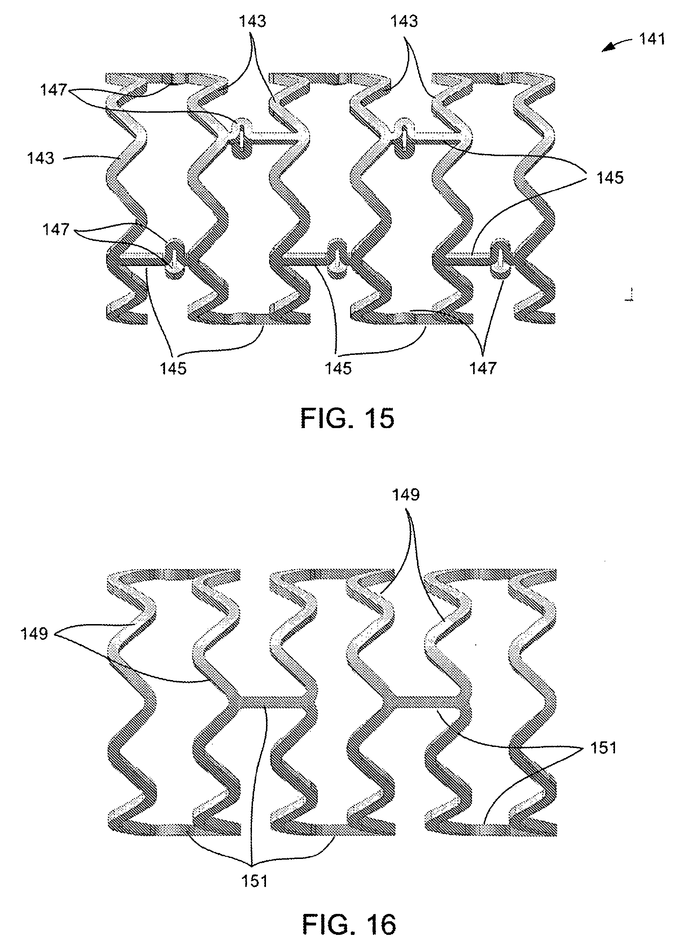

Examples of some optimized stent designs which take advantage of the inherent material properties of the formed polymeric substrate are illustrated in the side views of Figs. 15 and 16. Such designs are particularly optimized for forming stents utilizing materials such as PLLA having the relatively high molecular weight described herein with a crystallinity of, e.g., 20% - 40%. Such a stent may be utilized in a region of a patient's body which is subjected to high dynamic forces, such as the SFA, as discussed above. As discussed above, high molecular weight PLLA may have an elastic recoil ranging from, e.g., 0% to 4%, and stent designs as shown may typically experience physiologic conditions which induce material strain of less than 5% in axial, radial, and bending modes.

The stent designs may also accommodate relatively high levels of deformation in a variety of modes (radial, axial, bending, etc) while staying within, e.g., a 150% material strain limit, of various substrate materials. Examples of such high strain situations include crushing, shortening, stretching, and bending of the stent due to motion and external forces. The stent designs thus allow the stent to withstand such motion without fracturing by maintaining material strain below the ultimate strain of the material.

As shown in the side view of Fig. 15, stent 141 may include a number of undulating circumferential support element 143 which are coupled to one another via one or more linking or coupling elements 145. Although illustrated with six support elements **143,** the number of support elements **143** may be varied depending upon the desired length of the overall stent **141** to be implanted. The support elements **143** may form an undulating wave which are coupled by one or more, e.g., three, linking or coupling elements **145,** which are aligned in parallel and uniformly and circumferentially spaced apart relative to one another with respect to a longitudinal axis defined by the stent **141.** The coupling elements **145** may incorporate or define a curved or arcuate section **147** along its length where the section **147** defines a radius which is smaller than a radius defined by the undulating portions of support elements **143.** These curved or arcuate sections **147** may serve a stress-relief function in the event that the stent **141** has a longitudinal force imparted upon the stent **141.**

Another variations is illustrated in the side view of Fig. 16, which similarly shows one or more undulating circumferential support element **149,** e.g., six support elements **149,** which are similarly connected by one or more linking or coupling elements **151.** In this example, two linking or coupling elements **151** which are apposed to one another along a circumference of support element **149** may connect or attach adjacent support elements **149** to one another. Each adjacent support element **149** may be coupled via the linking or coupling elements **151** aligned in an alternating pattern to provide the overall stent with sufficient flexibility along its length.

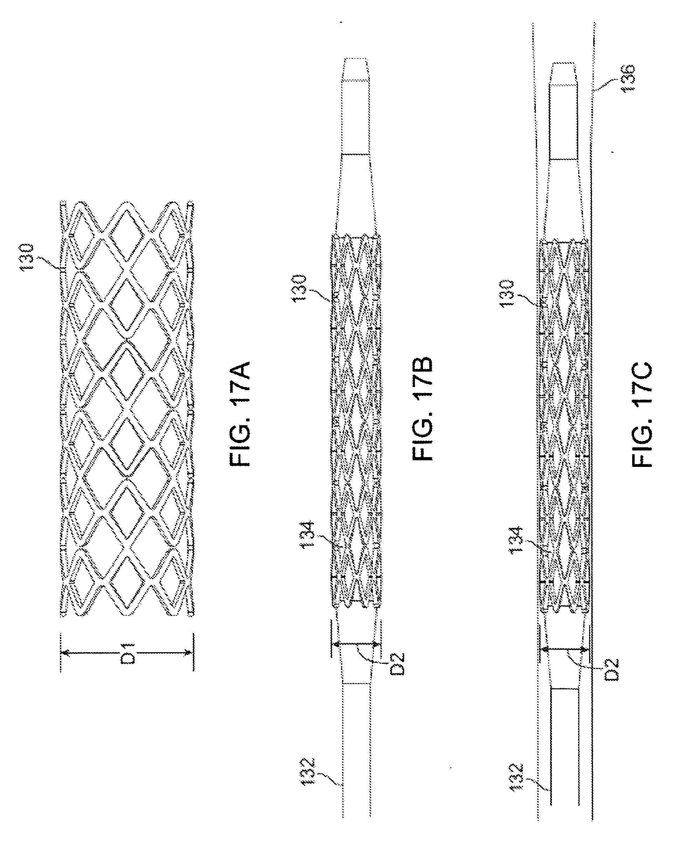

Figs. 17A to 17F illustrate side views of another example of how a stent **130** formed from a polymeric substrate may be delivered and deployed for secure expansion within a vessel. Fig. 17A shows a side view of an exemplary stent **130** which has been processed or cut from a polymeric substrate formed with an initial diameter **D1.** As described above, the substrate may be heat treated at, near, or above the glass transition temperature T_{g} of the substrate to set this initial diameter **D1** and the substrate may then be processed to produce the stent **130** such that the stent **130** has a corresponding diameter **D1.** Stent **130** may then be reduced in diameter to a second delivery diameter **D2** which is less than the initial diameter **D1** such that the stent **130** may be positioned upon, e.g., an inflation balloon **134** of a delivery catheter **132,** as shown in Fig. 17B. The stent **130** at its reduced diameter **D2** may be self-constrained such that the stent **130** remains in its reduced diameter **D2** without the need for an outer sheath, although a sheath may be optionally utilized. Additionally, because of the processing and the resultant material characteristics of the stent material, as described above, the stent **130** may be reduced from initial diameter **D1** to delivery diameter **D2** without cracking or material failure.

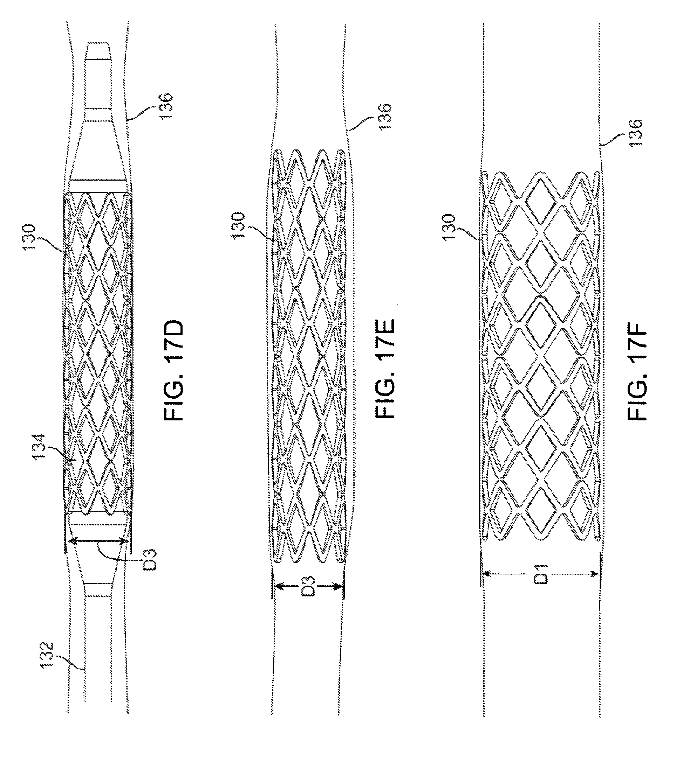

With stent **130** positioned upon delivery catheter **132,** it may be advanced intravascularly within a vessel **136** until the delivery site is reached, as shown in Fig. 17C. Inflation balloon **134** may be inflated to expand a diameter of stent **130** into contact against the vessel interior, e.g., to an intermediate diameter **D3,** which is less than the stent's initial diameter **D1** yet larger than the delivery diameter **D2.** Stent **130** may be expanded to this intermediate diameter **D3,** as shown in Fig. 17D, without any cracking or failure because of the inherent material characteristics described above. Moreover, expansion to intermediate diameter **D3** may allow for the stent **130** to securely contact the vessel wall while allowing for the withdrawal of the delivery catheter **132,** as shown in Fig. 17E.

Once the stent **130** has been expanded to some intermediate diameter **D3** and secured against the vessel wall, stent **130** may be allowed to then self-expand further over a period of time into further contact with the vessel wall such that stent **130** conforms securely to the tissue. This self-expansion feature ultimately allows for the stent **130** to expand back to its initial diameter **D1** which had been heat set, as shown in Fig. 17F, or until stent **130** has fully self-expanded within the confines of the vessel diameter.

These examples are presented to be illustrative of the types of devices which may be formed and various other devices which may be formed from the polymeric substrate are also included within this disclosure.

The applications of the disclosed invention discussed above are not limited to certain processes, treatments, or placement in certain regions of the body, but may include any number of other processes, treatments, and areas of the body. Modification of the above-described methods and devices for carrying out the invention, and variations of aspects of the invention that are obvious to those of skill in the arts are intended to be within the scope of this disclosure.

## Claims

1. A method of forming a self-expanding stent scaffold from a polymeric substrate, comprising:
immersing a mandrel (40) into at least a first polymeric solution (46) such that at least a first layer of a biocompatible polymer substrate is formed upon the mandrel and has a first diameter defined by the mandrel;
curing the substrate at a temperature above the glass transition temperature (Tg) of the substrate;
forming an expandable stent scaffold having the first diameter from the substrate;
reducing the first diameter of the stent to a second smaller diameter; and
quenching or cooling to a temperature below the glass transition temperature (Tg);
wherein the stent retains one or more mechanical properties of the polymer resin such that the stent exhibits ductility upon application of a load and a shape memory effect.

2. The method of claim 1, wherein prior to forming the expandable stent scaffold (130), the method further comprises:
controlling a number of immersions of the mandrel (40) into the first polymeric solution (46);
controlling a duration of time of each immersion of the mandrel; and
controlling a delay time between each immersion of the mandrel.

3. The method of claim 2 further comprising controlling a withdrawal rate of the mandrel (40) from the first polymeric solution (46) after each immersion.

4. The method of claim 1 wherein the first polymeric solution (46) is selected from the group consisting of polyethylene, polycarbonates, polyamides, polyesteramides, polyetheretherketone, polyacetals, poiyketals, polyurethane, polyolefin, polyethylene terephthalate, polylactide, poly-L-lactide, poly-glycolide, poly(lactide-co-glycolide), polycaprolactone, caprolactones, polydioxanones, polyanhydrides, polyorthocarbonates, polyphosphazenes, chitin, chitosan, poly(ainino acids), polyorthoesters, oligomers, homopolymers, methyl cerylate, methyl niethacrylate, acryli acid, methacrylic acid, acrylamide, hydroxyethy acrylate, hydroxyethyl methacrylate, glyceryl scrylate, glyceryl methacrylate, methacrylamide, ethacrylamide, styrene, vinyl chloride, binaly pyrrolidone, polyvinyl alcohol, polycoprolactarn, polylauryl lactam, polyjexamethylene adipamide, polyexamethyleiie dodecaoediamide, trimethylene carbonate, poly(β-hydroxybutyrate), poly(g-ethyl glutamate), poly(DTH iminocarbonate), poly(bisphenol A iminocarbonate), polycyanoacrylate, polyphosphazene, methyl cerylate, methyl niethacrylate, acryli acid, methacrylic acid, acrylamide, hydroxyethy acrylate, hydroxyethyl niethacrylate, glyceryl scrylate, glyceryl methacrylate, methacrylamide, ethacrylamide , and copolymers, terpolymers and combinations and mixtures thereof.

5. The method of claim 1 wherein immersing the mandrel (40) comprises further immersing the mandrel into a second polymeric solution such that a second layer of polymer is formed upon the first layer,

6. The method of claim 5 wherein the second polymeric solution comprises a drug or agent selected from the group consisting of antipoliferative, antineoplastic, antigenic, antiinflammatory, antirestenotic, antilipid, antimitotics, metalloproteinase inhabitors, and anti- selerosing agents.

7. The method of claim 5 wherein the second polymeric solution comprises a radio- opaque material.

8. The method of claim 7 wherein the mandrel (40) is immersed at one or both ends of the substrate into the radio-opaque material.

9. The method of claim 5 wherein the second polymeric solution comprises a BaSO₄ solution.

10. The method of claim 2 wherein controlling the number of immersions comprises immersing the mandrel (40) between 2 and 20 times.

11. The method of claim 2 wherein controlling the duration of time comprises immersing the mandrel (40) between 15 seconds and 240 minutes.

12. The method of claim 2 wherein controlling the delay time comprises delaying immersion of the mandrel (40) between 15 seconds and 60 minutes.

13. The method of claim 1 wherein immersing the mandrel (40) comprises inserting the mandrel into the first polymeric solution (46) at a rate of 5 mm min to 1000 mm/min.

14. The method of claim 1 further comprising rotating the mandrel (40) while applying heat.

15. The method of claim 1 further comprising controlling an angle of the mandrel (40) relative to the polymeric solution (46).

16. The method of claim 15 wherein controlling an angle comprises alternating an angle of the mandrel (40) up to 180 degrees

17. The method of claim 1 wherein the substrate has a length of 1 cm to 40 cm.

18. The method of claim 1 further comprising controlling a relative humidity of less than 30%.

19. The method of claim 1 further comprising controlling a temperature of the polymeric solution (46) to be below a boiling point of a solvent contained within the polymeric solution.

20. The method of claim 1 further comprising mold-transferring one or more patterns from an outer surface of the mandrel (46) to an inner surface of the substrate.

21. The method of claim 1 further comprising controlling a crystallinity percentage of the substrate.

22. The method of claim 1 wherein the substrate formed upon the mandrel (40) is isotropic.

23. The method of claim 1 wherein the expandable stent scaffold (130) exhibits a 20% radial deformation when placed under a 0.1 N to 20 N load,

24. The method of claim 1 wherein the expandable stent scaffold (130) exhibits a percent reduction in diameter of between 5% to 70% without fracture formation when placed under a compressive load.

25. The method of claim 1 wherein forming the expandable stent scaffold (130) comprises machining the stent from the substrate.

26. The method of claim 25 wherein the stent is adapted to curve up to 180 degrees about a 1 cm curvature radius without fracture formation.

27. The method of claim 25 wherein the stent is adapted to exhibit a percent reduction in axial length of between 10% to 30% without fracture formation when placed under an axial load.

28. The method of claim 1 wherein the first polymeric solution (46) comprises a polymer having a molecular weight ranging from 259,000 g/mol to 2,120,000 g/mol.

## Patentansprüche

1. Verfahren zum Bilden eines selbst weitenden Stentstützgerüstes aus einem Polymersubstrat, umfassend:
Eintauchen eines Dorns (40) in mindestens eine erste Polymerlösung (46), so dass mindestens eine erste Schicht aus einem biokompatiblen Polymersubstrat auf dem Dorn gebildet wird und einen durch den Dorn festgelegten ersten Durchmesser aufweist;
Härten des Substrats bei einer Temperatur über der Glasübergangstemperatur (Tg) des Substrats;
Bilden des weitbaren Stentstützgerüstes mit dem ersten Durchmesser aus dem Substrat;
Reduzieren des ersten Durchmessers des Stents auf einen zweiten kleineren Durchmesser; und
Quenchen oder Kühlen auf eine Temperatur unter der Glasübergangstemperatur (Tg);
wobei der Stent eine oder mehrere mechanische Eigenschaften des Polymerharzes beibehält, so dass der Stent bei Anlegen einer Last Duktilität und eine Formgedächtniswirkung aufweist.

2. Verfahren nach Anspruch 1, wobei vor dem Bilden des weitbaren Stentstützgerüstes (130) das Verfahren weiterhin umfasst:
Steuern einer Anzahl von Eintauchvorgängen des Dorns (40) in die erste Polymerlösung (46);
Steuern einer Zeitdauer jedes Eintauchvorgangs des Dorns; und
Steuern einer Verzögerungszeit zwischen jedem Eintauchen des Dorns.

3. Verfahren nach Anspruch 2, welches weiterhin das Steuern einer Entnahmegeschwindigkeit des Dorns (40) aus der ersten Polymerlösung (46) nach jedem Eintauchen umfasst.

4. Verfahren nach Anspruch 1, wobei die erste Polymerlösung (46) aus der Gruppe bestehend aus Polyethylen, Polycarbonaten, Polyamiden, Polyesteramiden, Polyetheretherketon, Polyacetalen, Polyketalen, Polyurethan, Polyolefin, Polyethylenterephthalat, Polylactid, Poly-L-lactid, Polyglycolid, Poly(lactid-co-glycolid), Polycaprolacton, Caprolactonen, Polydioxanonen, Polyanhydriden, Polyorthocarbonaten, Polyphosphazenen, Chitin, Chitosan, Poiy(aininosäuren), Polyorthoestern, Oligomeren Homopolymeren, Methylcerylat, Methylniethacrylat, Acrylsäure, Methacrylsäure, Acrylamid, Hydroxyethyacrylat, Hydroxyethylmethacrylat, Glycerylscrylat, Glycerylmethacrylat, Methacrylamid, Ethacrylamid, Styren, Vinylchlorid, Binaly- Pyrrolidon, Polyvinylalkohol, Polycoprolactarn, Polylauryllactam, Polyjexamethylen-Adipamid, Polyexamethyleiie-Dodecaoediamid, Trimethylencarbonat, Poly(ß-hydroxybutyrat), Poly(g-ethylglutamat), Poly(DTH-Iminocarbonat), Poly(bisphenol A Iminocarbonat), Polycyanoacrylat, Polyphosphazen, Methylcerylat, Methylniethacrylat, Acrylsäure, Methacrylsäure, Acrylamid, Hydroxyethyacrylat, Hydroxyethylniethacrylat, Glycerylscrylat, Glycerylmethacrylat, Methacrylamid, Ethacrylamid und Copolymeren, Terpolymeren und Kombinationen und Mischungen derselben gewählt wird.

5. Verfahren nach Anspruch 1, wobei das Eintauchen des Dorns (40) weiteres Eintauchen des Dorns in eine zweite Polymerlösung umfasst, so dass eine zweite Polymerschicht auf der ersten Schicht gebildet wird.

6. Verfahren nach Anspruch 5, wobei die zweite Polymerlösung ein Arzneimittel oder einen Wirkstoff gewählt aus der Gruppe bestehend aus antiproliferativen, antineoplastischen, antigenen, entzündungshemmenden, antirestenotischen, Antilipid-, antimitotischen, Metalloproteinase-Hemmer- und antisklerotischen Wirkstoffen umfasst.

7. Verfahren nach Anspruch 5, wobei die zweite Polymerlösung ein radioopakes Material umfasst.

8. Verfahren nach Anspruch 7, wobei der Dorn (40) an einem oder an beiden Enden des Substrats in das radioopake Material eingetaucht wird.

9. Verfahren nach Anspruch 5, wobei die zweite Polymerlösung eine BaSO₄-Lösung umfasst.

10. Verfahren nach Anspruch 2, wobei das Steuern der Anzahl von Eintauchvorgängen das zwei- bis zwanzigmalige Eintauchen des Dorns (40) umfasst.

11. Verfahren nach Anspruch 2, wobei das Steuern der Zeitdauer das Eintauchen des Dorns (40) zwischen 15 Sekunden und 240 Minuten umfasst.

12. Verfahren nach Anspruch 2, wobei das Steuern der Verzögerungszeit das Verzögern des Eintauchens des Dorns (40) zwischen 15 Sekunden und 60 Minuten umfasst.

13. Verfahren nach Anspruch 1, wobei das Eintauchen des Dorns (40) das Einführen des Dorns in die erste Polymerlösung (46) bei einer Geschwindigkeit von 5 mm min bis 1000 mm/min umfasst.

14. Verfahren nach Anspruch 1, welches weiterhin das Drehen des Dorns (40) während eines Anlegens von Wärme umfasst.

15. Verfahren nach Anspruch 1, welches weiterhin das Steuern eines Winkels des Dorns (40) relativ zu der Polymerlösung (46) umfasst.

16. Verfahren nach Anspruch 15, wobei das Steuern eines Winkels das Wechseln eines Winkels des Dorns (40) um bis zu 180 Grad umfasst.

17. Verfahren nach Anspruch 1, wobei das Substrat eine Länge von 1 cm bis 40 cm hat.

18. Verfahren nach Anspruch 1, welches weiterhin das Steuern einer relativen Feuchte von weniger als 30% umfasst.

19. Verfahren nach Anspruch 1, welches weiterhin das Steuern einer Temperatur der Polymerlösung (46) unter einen Siedepunkt eines Lösungsmittels, das in der Polymerlösung enthalten ist, umfasst.

20. Verfahren nach Anspruch 1, welches weiterhin das Formübertragen von einem oder mehreren Mustern von einer Außenfläche des Dorns (46) auf eine Innenfläche des Substrats umfasst.

21. Verfahren nach Anspruch 1, welches weiterhin das Steuern eines Kristallinitätsprozentsatzes des Substrats umfasst.

22. Verfahren nach Anspruch 1, wobei das auf dem Dorn (40) ausgebildete Substrat isotrop ist.

23. Verfahren nach Anspruch 1, wobei das weitbare Stentstützgerüst (130) eine radiale Verformung von 20% aufweist, wenn es einer Last von 0,1 N bis 20 N ausgesetzt wird.

24. Verfahren nach Anspruch 1, wobei das weitbare Stentstützgerüst (130) bei Druckbelastung ohne Rissbildung eine prozentuale Durchmesserreduktion von 5% bis 70% aufweist.

25. Verfahren nach Anspruch 1, wobei das Bilden des weitbaren Stentstützgerüstes (130) das maschinelle Herstellen des Stents aus dem Substrat umfasst.

26. Verfahren nach Anspruch 25, wobei der Stent ausgelegt ist, um sich ohne Rissbildung bis zu 180 Grad um einen Krümmungsradius von 1 cm zu biegen.

27. Verfahren nach Anspruch 25, wobei der Stent ausgelegt ist, um bei axialer Belastung ohne Rissbildung eine prozentuale Reduzierung der axialen Länge von 10% bis 30% aufzuweisen.

28. Verfahren nach Anspruch 1, wobei die erste Polymerlösung (46) ein Polymer mit einer relativen Molekülmasse, die von 259.000 g/mol bis 2.120.000 g/mol reicht, umfasst.

## Revendications

1. Procédé de formation d'un échafaudage de stent auto-extensible à partir d'un substrat polymère, comprenant :
l'immersion d'un mandrin (40) dans au moins une première solution polymère (46) de telle sorte qu'au moins une première couche d'un substrat polymère biocompatible soit formée sur le mandrin et ait un premier diamètre défini par le mandrin ;
le durcissement du substrat à une température supérieure à la température de transition vitreuse (Tg) du substrat ;
la formation d'un échafaudage de stent extensible ayant le premier diamètre à partir du substrat ;
la réduction du premier diamètre du stent à un second diamètre plus petit ; et
le refroidissement rapide ou refroidissement à une température en dessous de la température de transition vitreuse (Tg) ;
dans lequel le stent conserve une ou plusieurs propriétés mécaniques de la résine polymère de telle sorte que le stent présente une certaine ductilité lors de l'application d'une charge et un effet de mémoire de forme.

2. Procédé selon la revendication 1, dans lequel, avant la formation de l'échafaudage de stent extensible (130), le procédé comprend en outre :
le contrôle d'un nombre d'immersions du mandrin (40) dans la première solution polymère (46) ;
le contrôle d'une durée de chaque immersion du mandrin ; et
le contrôle d'un délai d'attente entre chaque immersion du mandrin.

3. Procédé selon la revendication 2, comprenant en outre le contrôle d'une vitesse de retrait du mandrin (40) de la première solution polymère (46) après chaque immersion.

4. Procédé selon la revendication 1, dans lequel la première solution polymère (46) est choisie dans le groupe constitué de polyéthylène, polycarbonates, polyamides, polyesteramides, polyétheréthercétone, polyacétals, polycétals, polyuréthane, polyoléfine, téréphtalate de polyéthylène, polylactide, poly-L-lactide, poly-glycolide, poly(lactide-co-glycolide), polycaprolactone, caprolactones, polydioxanones, polyanhydrides, polyorthocarbonates, polyphosphazènes, chitine, chitosane, acides polyaminés, polyorthoesters, oligomères, homopolymères, cérylate de méthyle, méthacrylate de méthyle, acide acrylique, acide méthacrylique, acrylamide, acrylate d'hydroxyéthyle, méthacrylate d'hydroxyéthyle, acrylate de glycéryle, méthacrylate de glycéryle, méthacrylamide, éthacrylamide, styrène, chlorure de vinyle, pyrrolidone binaire, alcool polyvinylique, polycaprolactame, polylactame de lauryle, polyhexaméthylène adipamide, polyhexaméthylène dodécanediamide, carbonate de triméthylène, poly(β-hydroxybutyrate), poly(g-éthyl glutamate), poly(DTH iminocarbonate), poly(bisphénol A iminocarbonate), polycyanoacrylate, polyphosphazène, cérylate de méthyle, méthacrylate de méthyle, acide acrylique, acide méthacrylique, acrylamide, acrylate d'hydroxyéthyle, méthacrylate d'hydroxyéthyle, acrylate de glycéryle, méthacrylate de glycéryle, méthacrylamide, éthacrylamide et de copolymères, terpolymères et combinaisons et mélanges de ceux-ci.

5. Procédé selon la revendication 1, dans lequel l'immersion du mandrin (40) comprend en outre l'immersion du mandrin dans une seconde solution polymère de telle sorte qu'une seconde couche de polymère soit formée sur la première couche.

6. Procédé selon la revendication 5, dans lequel la seconde solution polymère comprend un médicament ou agent choisi dans le groupe constitué d'agents antiprolifératifs, antinéoplasiques, antigéniques, anti-inflammatoires, anti-resténose, anti-lipide, antimitotiques, inhibiteurs de métalloprotéinases et anti-sclérosants.

7. Procédé selon la revendication 5, dans lequel la seconde solution polymère comprend une matière radio-opaque.

8. Procédé selon la revendication 7, dans lequel le mandrin (40) est immergé au niveau d'une ou des deux extrémités du substrat dans la matière radio-opaque.

9. Procédé selon la revendication 5, dans lequel la seconde solution polymère comprend une solution de BaSO₄.

10. Procédé selon la revendication 2, dans lequel le contrôle du nombre d'immersions comprend l'immersion du mandrin (40) entre 2 et 20 fois.

11. Procédé selon la revendication 2, dans lequel le contrôle de la durée comprend l'immersion du mandrin (40) entre 15 secondes et 240 minutes.

12. Procédé selon la revendication 2, dans lequel le contrôle du délai d'attente comprend l'attente de l'immersion du mandrin (40) entre 15 secondes et 60 minutes.

13. Procédé selon la revendication 1, dans lequel l'immersion du mandrin (40) comprend l'insertion du mandrin dans la première solution polymère (46) à une vitesse de 5 mm/min à 1000 mm/min.

14. Procédé selon la revendication 1, comprenant en outre la rotation du mandrin (40) tout en appliquant de la chaleur.

15. Procédé selon la revendication 1, comprenant en outre le contrôle d'un angle du mandrin (40) par rapport à la solution polymère (46).

16. Procédé selon la revendication 15, dans lequel le contrôle d'un angle comprend la variation d'un angle du mandrin (40) jusqu'à 180 degrés.

17. Procédé selon la revendication 1, dans lequel le substrat a une longueur de 1 cm à 40 cm.

18. Procédé selon la revendication 1, comprenant en outre le contrôle d'une humidité relative inférieure à 30 %.

19. Procédé selon la revendication 1, comprenant en outre le contrôle d'une température de la solution polymère (46) à une valeur inférieure à un point d'ébullition d'un solvant contenu à l'intérieur de la solution polymère.

20. Procédé selon la revendication 1, comprenant en outre le moulage par transfert d'un ou plusieurs motifs d'une surface externe du mandrin (46) à une surface interne du substrat.

21. Procédé selon la revendication 1, comprenant en outre le contrôle d'un pourcentage de cristallinité du substrat.

22. Procédé selon la revendication 1, dans lequel le substrat formé sur le mandrin (40) est isotrope.

23. Procédé selon la revendication 1, dans lequel l'échafaudage de stent extensible (130) présente une déformation radiale de 20 % lorsqu'il est placé sous une charge de 0,1 N à 20 N.

24. Procédé selon la revendication 1, dans lequel l'échafaudage de stent extensible (130) présente une réduction de diamètre en pourcentage entre 5 % et 70 % sans formation de fracture lorsqu'il est placé sous une charge de compression.

25. Procédé selon la revendication 1, dans lequel la formation de l'échafaudage de stent extensible (130) comprend l'usinage du stent à partir du substrat.

26. Procédé selon la revendication 25, dans lequel le stent est conçu pour s'incurver jusqu'à 180 degrés sur un rayon de courbure d'environ 1 cm sans formation de fracture.

27. Procédé selon la revendication 25, dans lequel le stent est conçu pour présenter une réduction en pourcentage de longueur axiale entre 10 % et 30 % sans formation de fracture lorsqu'il est placé sous une charge axiale.

28. Procédé selon la revendication 1, dans lequel la première solution polymère (46) comprend un polymère ayant une masse moléculaire allant de 259 000 g/mole à 2 120 000 g/mole.
